(19) Europäisches Patentamt
European Patent Office
Office européen des brevets

(11) **EP 3 609 994 B1**

(12) **FASCICULE DE BREVET EUROPEEN**

(45) Date de publication et mention de la délivrance du brevet:
**14.06.2023 Bulletin 2023/24**

(21) Numéro de dépôt: **18720318.7**

(22) Date de dépôt: **10.04.2018**

(51) Classification Internationale des Brevets (IPC):
**B01L 3/00** *(2006.01)*

(52) Classification Coopérative des Brevets (CPC):
**B01L 3/508; C12M 23/10; C12M 25/02; C12M 33/14; C12Q 1/04; C12Q 1/06;**
B01L 2200/0621; B01L 2200/0689; B01L 2300/0681; B01L 2300/0832; B01L 2400/049; B01L 2400/0644

(86) Numéro de dépôt international:
**PCT/FR2018/050902**

(87) Numéro de publication internationale:
**WO 2018/189478 (18.10.2018 Gazette 2018/42)**

(54) **DISPOSITIF DE CONTROLE MICROBIOLOGIQUE, PROCEDE DE MISE A DISPOSITION ET UTILISATION D'UN TEL DISPOSITIF**

MIKROBIOLOGISCHE TESTVORRICHTUNG, VERFAHREN ZUR BEREITSTELLUNG UND VERWENDUNG EINER SOLCHEN VORRICHTUNG

MICROBIOLOGICAL TESTING DEVICE, METHOD FOR PROVISION AND USE OF SUCH A DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.04.2017 FR 1753250**

(43) Date de publication de la demande:
**19.02.2020 Bulletin 2020/08**

(73) Titulaire: **Biomérieux**
**69280 Marcy l'Étoile (FR)**

(72) Inventeurs:
• **MICHEL, Florian**
**42400 Saint-Chamond (FR)**
• **FOUCAULT, Frédéric**
**69280 Marcy L'Etoile (FR)**
• **ROZAND, Christine**
**69290 Saint-Genis-les-Ollières (FR)**

(74) Mandataire: **bioMérieux PI Groupement mandataires**
**bioMérieux**
**69280 Marcy l'Etoile (FR)**

(56) Documents cités:
FR-A- 1 138 452     GB-A- 2 268 187
US-A- 2 677 646     US-A- 3 539 300
US-A1- 2014 342 447     US-A1- 2015 072 377
US-A1- 2016 348 055

**Description**

La présente invention concerne, de façon générale, le domaine de l'analyse microbiologique. Plus particulièrement, elle porte sur un dispositif de contrôle microbiologique pour contrôler un liquide à analyser, ce liquide étant susceptible de contenir au moins un micro-organisme. Elle porte aussi sur un procédé de mise à disposition d'un tel dispositif et sur des utilisations d'un tel dispositif dans un procédé de contrôle d'un liquide à analyser susceptible de contenir au moins un micro-organisme.

L'invention vise plus particulièrement le domaine du contrôle microbiologique industriel agroalimentaire, pharmaceutique ou cosmétique.

L'invention a été développée suite à de travaux ayant bénéficié de la participation du Centre National d'Etudes Spatiales (CNES).

Il existe de nombreuses situations dans lesquelles on cherche à contrôler la présence d'au moins un micro-organisme dans un liquide, en vue généralement de pouvoir constater l'absence de ce micro-organisme.

Il est connu du document US2014342447A1, un dispositif de culture cellulaire comprenant un logement qui contient un couvercle ayant une fenêtre optiquement transparente (le couvercle peut ou non être amovible) ; un canal de distribution de fluide, par exemple un canal unique ou connecté à une pluralité de canaux ; un orifice d'injection d'échantillon relié fluidiquement au canal de distribution de fluide ; une base logeant un tampon de support poreux ; et un orifice d'injection de support relié fluidiquement au tampon de support. Le couvercle s'accouple à la base pour former un joint stérile ; le canal de distribution de fluide est disposé sur le tampon de support, qui est visible à travers la fenêtre optique ; et l'échantillon de fluide introduit dans le canal de distribution de fluide est réparti uniformément sur le tampon de milieu, par exemple via une pluralité de canaux.

Bien entendu, le liquide à analyser peut-être un fluide biologique (sang total, sérum, plasma, urine, liquide céphalo-rachidien, sécrétion organique, etc....)- Toutefois, le liquide peut aussi être un liquide industriel, notamment un liquide alimentaire (eau, boisson en général et notamment jus de fruits, lait, soda, etc..) ou un liquide pharmaceutique ou cosmétologique.

De nombreuses techniques de laboratoire sont connues qui permettent de filtrer le liquide à analyser pour recueillir d'éventuels micro-organismes contenus dans le liquide, de mettre en culture ces microorganismes pour être ensuite en mesure de les détecter, de les dénombrer, de les caractériser et/ou de les identifier. Ces techniques requièrent un certain nombre de manipulations bien connues des laborantins.

Dans ces techniques, on est parfois amené à utiliser un dispositif de filtration qui comprend un espace interne fermé délimité par une enceinte et qui est destiné à recevoir le liquide à analyser. Une telle technique est notamment appelée « filtration sur membrane ». Un moyen de filtration microbiologique, par exemple une membrane filtrante, est disposé dans l'espace interne fermé et sépare, dans l'espace interne fermé, un premier compartiment d'un second compartiment de l'espace interne fermé. Le dispositif comporte un port d'entrée pour le liquide à analyser qui débouche dans le premier compartiment de l'espace interne fermé.

Dans les dispositifs de filtration connus, comme dans ceux du document EP-1.783.494, il est prévu un port d'aspiration qui est destiné à être relié à une source externe d'aspiration. Dans l'utilisation d'un tel dispositif, on doit donc raccorder la source externe d'aspiration au port d'aspiration pour générer, lors de l'introduction du liquide à analyser dans le dispositif par le port d'entrée, une dépression à l'intérieur de l'espace interne fermé, cette dépression étant favorable, voire nécessaire, à la filtration. Une fois la filtration réalisée, les micro-organismes étant retenus sur le moyen de filtration, le dispositif est ouvert pour récupérer le moyen de filtration, lequel est transféré dans un dispositif de mise en culture pour permettre l'incubation des micro-organismes.

Une telle technique est aisément réalisable en laboratoire.

En revanche, une telle technique est difficilement mise en oeuvre dans un milieu opérationnel, notamment dans un milieu industriel dans lequel les liquides sont produits, conditionnés, distribués ou utilisés. En effet, dans ce contexte, il est intéressant de pouvoir disposer de moyens pour détecter une éventuelle contamination du liquide par un micro-organisme non souhaité. Cependant, les techniques habituelles telles que décrites ci-dessus nécessitent de transporter un échantillon du liquide à analyser vers un laboratoire où les méthodes habituelles peuvent être mises en oeuvre. Il est en effet peu envisageable de réaliser ces opérations habituelles in situ, sur le site industriel de production, conditionnement, distribution ou utilisation du liquide. En effet la manipulation d'un liquide contaminé dans un tel environnement poserait le risque d'une propagation de la contamination en cas de mauvaise manipulation. Par ailleurs, l'étape de mise en culture des éventuels microorganismes nécessite la présence d'un milieu nutritionnel qui est, par définition, favorable au développement des micro-organismes. On ne souhaite bien entendu pas qu'un tel milieu nutritionnel soit introduit dans un tel contexte industriel. De plus, les techniques habituelles de détection nécessitent aussi de protéger l'échantillon à analyser de toute contamination externe, donc de travailler dans un environnement le plus stérile possible pour éviter les faux positifs.

L'invention a donc pour but de proposer un dispositif et un procédé de contrôle microbiologique d'un liquide à analyser qui permettent des opérations de contrôle particulièrement simplifiées, qu'il serait même envisageable d'utiliser ou de conduire en dehors du laboratoire microbiologique, y compris dans un environnement industriel.

Dans ce but, l'invention propose tout d'abord un dispositif de contrôle microbiologique pour contrôler un liquide à analyser susceptible de contenir au moins un micro-organisme, comportant, dans une configuration de mise à disposition dudit dispositif de contrôle microbiologique avant utilisation :

- un espace interne fermé délimité par une enceinte et destiné à recevoir le liquide à analyser
- un moyen de filtration microbiologique disposé dans l'espace interne fermé et séparant, dans l'espace interne fermé, un premier compartiment d'un second compartiment de l'espace interne fermé ;
- un port d'entrée pour le liquide à analyser, le port d'entrée débouchant dans le premier compartiment de l'espace interne fermé.

Un tel dispositif est caractérisé en ce que le dispositif de contrôle microbiologique comporte, à l'intérieur de l'espace interne fermé, une couche nutritive comprenant une composition d'un milieu de culture microbiologique, la couche nutritive étant en contact avec le moyen de filtration, en ce que le port d'entrée du dispositif de contrôle microbiologique comporte un obturateur, et en ce que, dans la configuration de mise à disposition du dispositif de contrôle microbiologique avant utilisation :

- l'obturateur du port d'entrée est dans un état fermé pour fermer le port d'entrée et l'espace interne fermé de manière étanche à l'air, l'espace interne fermé étant isolé de toute source externe d'aspiration ;
- la pression gazeuse absolue à l'intérieur de l'espace interne fermé, ramenée à une température de 25°C, est strictement inférieure à la pression atmosphérique standard de 100 kPa à 25°C, de manière que le dispositif est apte à créer une aspiration au travers du port d'entrée lors d'une première ouverture de l'obturateur.

Selon d'autres caractéristiques optionnelles d'un dispositif selon l'invention, prises seules ou en combinaison :

- Le second compartiment de l'espace interne fermé est dépourvu de port de communication fluidique avec l'extérieur de l'espace interne fermé.
- Dans la configuration de mise à disposition du dispositif avant utilisation, le milieu de culture microbiologique de la couche nutritive est déshydraté.
- Tout échange fluidique entre le premier et le second compartiment de l'espace interne fermé se fait au travers du moyen de filtration.
- Le dispositif de contrôle microbiologique comporte un support pour le moyen de filtration et la couche nutritive.
- Le support pour le moyen de filtration comprend une grille, par exemple réalisée sous la forme d'une plaque perforée, qui s'étend en travers de l'espace interne fermé, entre le premier compartiment et le second compartiment.

- La couche nutritive est agencée entre le moyen de filtration et le support pour le moyen de filtration.
- La couche nutritive est serrée localement entre le moyen de filtration et le support pour le moyen de filtration.
- Le support pour le moyen de filtration comprend des cloisons de support agencées dans le second compartiment.
- Des cloisons de support sont ajourées pour permettre la circulation de fluide de part et d'autre desdites cloisons dans le second compartiment. - Un matériau absorbant l'eau est agencé dans le second compartiment.
- L'obturateur du port d'entrée comporte une vanne.
- L'obturateur du port d'entrée comporte une membrane étanche, et l'obturateur du port d'entrée est amené dans un état ouvert par rupture de la membrane.
- L'enceinte du dispositif de contrôle microbiologique comporte au moins un corps principal qui délimite au moins en partie le second compartiment, et comporte un couvercle qui délimite au moins en partie le premier compartiment, le corps principal et le couvercle étant formés de pièces distinctes assemblées l'une à l'autre pour former le dispositif de contrôle microbiologique.
- L'enceinte du dispositif de contrôle microbiologique comporte au moins une partie transparente.
- Le port d'entrée comporte un répartiteur comportant plusieurs passages distincts pour le liquide à analyser.
- Le port d'entrée comprend une portion interne qui débouche dans le premier compartiment de l'espace interne fermé, et une portion externe de raccordement à un contenant de liquide à analyser, et l'obturateur du port d'entrée est interposé entre la portion interne et la portion externe du port d'entrée.
- Dans la configuration de mise à disposition du dispositif de contrôle microbiologique avant utilisation, la pression gazeuse absolue à l'intérieur de l'espace interne fermé est telle qu'elle permet l'entrée d'un volume prédéterminé de l'échantillon à analyser sans évacuation de fluide depuis l'espace interne lors de l'entrée d'un volume prédéterminé de l'échantillon à analyser. Notamment, la pression gazeuse absolue à l'intérieur de l'espace interne fermé est de préférence strictement inférieure à la pression atmosphérique standard multipliée par le rapport du volume libre final dans l'espace interne, après l'entrée d'un volume prédéterminé de l'échantillon à analyser, divisé par le volume de l'espace interne. En pratique, dans la configuration de mise à disposition du dispositif de contrôle microbiologique avant utilisation, la pression gazeuse absolue à l'intérieur de l'espace interne fermé est strictement inférieure à 60 kPa absolus, de préférence strictement inférieure à 30 kPa absolus, plus préférentiellement strictement inférieure à 20 kPa absolus.

L'invention concerne aussi un procédé de mise à disposition d'un dispositif de contrôle microbiologique pour contrôler un liquide à analyser susceptible de contenir au moins un micro-organisme, du type comprenant la fourniture d'un dispositif de contrôle microbiologique comportant:

- une enceinte prévue pour délimiter un espace interne fermé destiné à recevoir le liquide à analyser ;
- un moyen de filtration microbiologique prévu pour être disposé dans l'espace interne fermé et pour séparer, dans l'espace interne fermé, un premier compartiment d'un second compartiment de l'espace interne fermé ;
- un port d'entrée pour le liquide à analyser, le port d'entrée débouchant dans le premier compartiment de l'espace interne fermé,

caractérisé en ce que le procédé comprend la fourniture d'une couche nutritive, prévue pour être reçue à l'intérieur de l'espace interne fermé et imprégnée d'une composition d'un milieu de culture microbiologique, la couche nutritive étant en contact avec le moyen de filtration, et en ce que le procédé comprend, avant tout raccordement du dispositif de contrôle microbiologique à un contenant de liquide à analyser, successivement et dans cet ordre :

- une étape de dépressurisation pour abaisser la pression gazeuse absolue à l'intérieur de l'espace interne fermé ;
- - une étape d'obturation pour fermer l'espace interne fermé de manière étanche à l'air.

L'invention concerne aussi l'utilisation d'un dispositif de contrôle microbiologique, ayant l'une quelconque des caractéristiques énoncées ci-dessus, dans un procédé de contrôle d'un liquide à analyser susceptible de contenir au moins un micro-organisme.

Cette utilisation peut en outre comporter les étapes consistant à :

- raccorder un contenant de liquide à analyser au port d'entrée ; - ouvrir l'obturateur du port d'entrée pour permettre le passage du liquide à analyser du récipient vers l'espace interne fermé. Elle peut comporter les étapes ultérieures consistant à :
- refermer l'obturateur du port d'entrée ;
- déconnecter le contenant de liquide à analyser ;
- faire incuber, dans le dispositif de contrôle microbiologique, un éventuel micro-organisme contenu initialement dans le liquide à analyser.

Elle peut comporter une étape encore ultérieure consistant à détecter, dénombrer, identifier et/ou caractériser visuellement un éventuel micro-organisme contenu initialement dans le liquide à analyser par vision au travers d'une portion transparente de l'enceinte du dispositif de contrôle microbiologique.
Diverses autres caractéristiques ressortent de la description faite ci-dessous en référence aux dessins annexés qui montrent, à titre d'exemples non limitatifs, des formes de réalisation de l'objet de l'invention.

La Figure 1 est une vue en perspective éclatée d'un premier exemple de réalisation d'un dispositif selon l'invention.
La Figure 2 est une vue en perspective du dispositif de la Fig. 1, assemblé.
La Figure 3 est une vue en coupe du dispositif de la Fig. 2, le plan de coupe étant illustré à la Fig. 1.
La Figure 4 est une vue en perspective, en vue de dessous, du couvercle du dispositif de la Fig. 1.
On a illustré sur les Figures 1 à 4 un exemple de réalisation d'un dispositif 10 de contrôle microbiologique pour contrôler un liquide à analyser, ledit liquide étant susceptible de contenir au moins un micro-organisme.
La Figure 5 est une vue en perspective éclatée d'un second exemple de réalisation d'un dispositif selon l'invention.
La Figure 6 est une vue en perspective, de dessous, du corps principal et du fond rapporté du second exemple de réalisation d'un dispositif selon l'invention. La Figure 7 est une vue en coupe du dispositif de la Fig. 5, le plan de coupe étant illustré à la Fig. 5.

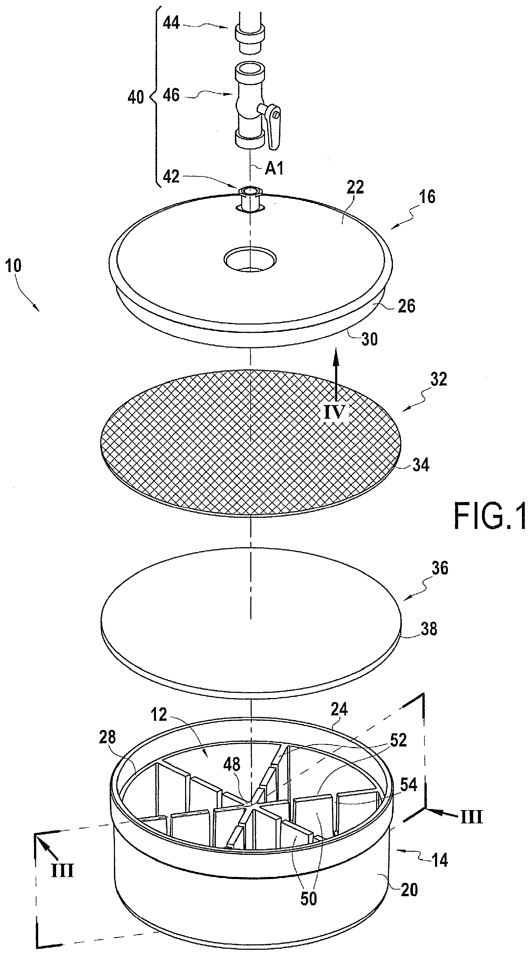

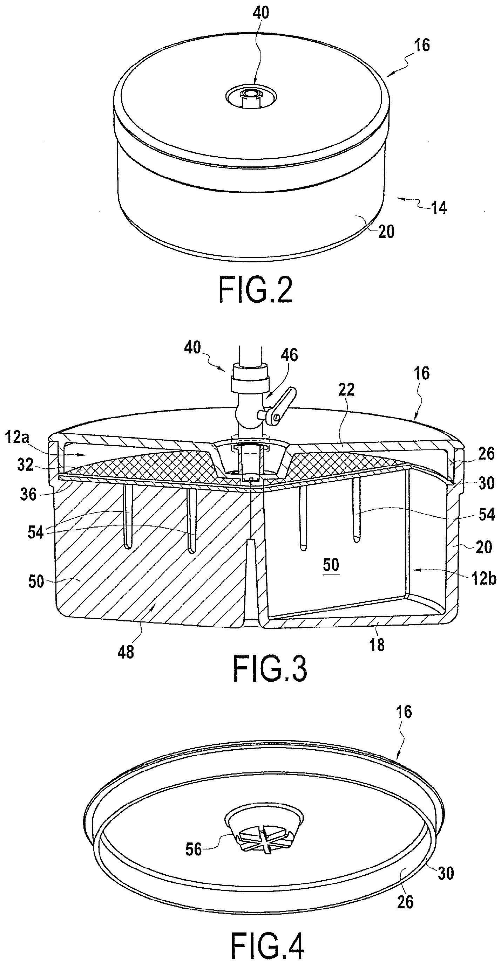

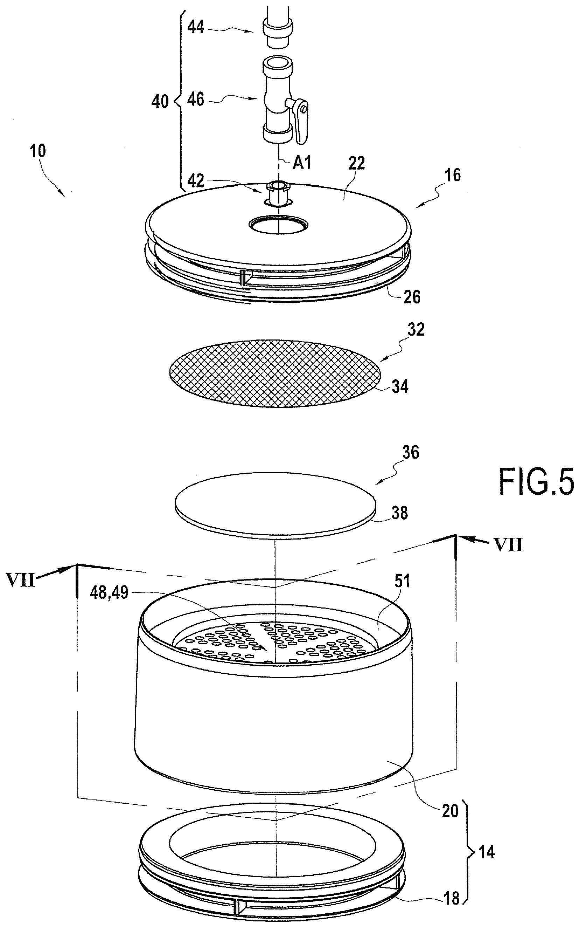

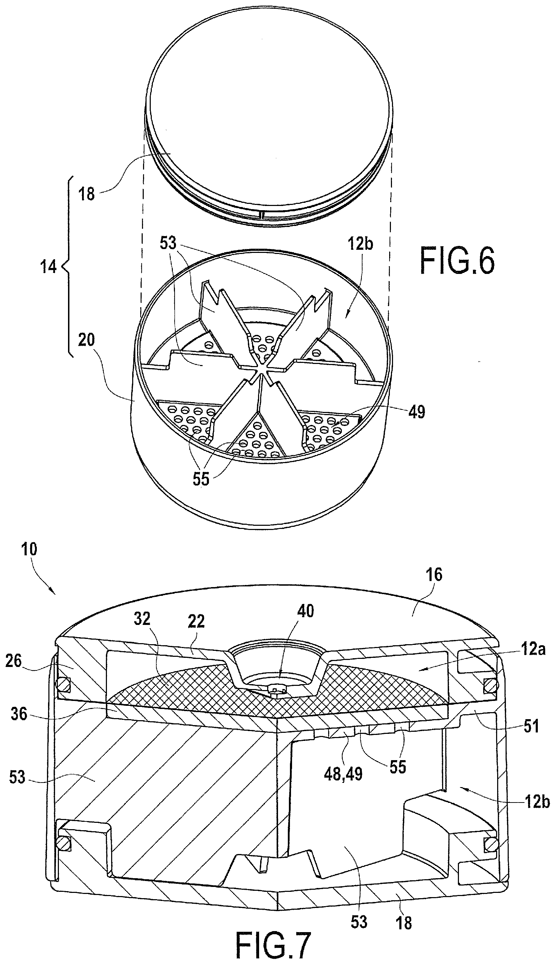

Au sens de la présente invention, le terme micro-organisme recouvre notamment les bactéries à Gram positif ou à Gram négatif, les levures, les amibes, les virus et plus généralement, les organismes unicellulaires, invisibles à l'œil nu, qui peuvent être manipulés et multipliés en laboratoire.
Selon un mode préféré de réalisation de l'invention, le micro-organisme est une bactérie, à Gram négative ou positive, ou une levure.
Ce dispositif de contrôle microbiologique, dont un premier exemple de réalisation est illustré aux Figs. 1 à 4 et un second exemple de réalisation est illustré aux Figs. 5 à 7, dans son état opérationnel illustré aux Figs. 2 et 3 pour le premier exemple mode de réalisation, et à la Fig. 7 pour le deuxième exemple mode de réalisation, présente un espace interne fermé 12, délimité par une enceinte et destiné à recevoir le liquide à analyser. Les deux exemples de réalisation illustrés seront pour l'essentiel décrits en même temps. Les caractéristiques qui les distinguent l'un de l'autre seront évoquées au fur et à mesure.
Dans les exemples illustrés, l'enceinte du dispositif de contrôle microbiologique comporte au moins un corps principal 14 et un couvercle 16. Le corps principal 14 et le couvercle 16 sont formés de pièces distinctes qui sont assemblées l'une à l'autre pour former l'enceinte du dispositif de contrôle microbiologique. Le couvercle 16 vient refermer le corps principal 14 pour délimiter l'espace interne fermé 12 du dispositif de contrôle microbiologique 10. Le couvercle 16 présente donc une forme complémentaire de celle du corps principal 14 pour assurer la fermeture de ce dernier.

Dans les exemples illustrés, le corps principal 14 présent une paroi de fond 18 et une paroi latérale périphérique 20 de sorte que le corps principal 14 est ouvert par une extrémité opposée à sa paroi de fond 18. Dans l'exemple illustré, la paroi latérale périphérique 20 présente un axe central A1. La paroi de fond 18 est dans le cas illustré une paroi transversale perpendiculaire à l'axe central A1 du corps principal 14. Dans le premier exemple de réalisation, la paroi de fond 18 est réalisée en une seule pièce avec la paroi latérale périphérique 20, tandis que dans le deuxième exemple de réalisation, la paroi de fond 18 est réalisée sous la forme d'une pièce distincte qui vient refermer, vers le bas, l'espace interne fermé 12. Dans le cas où la paroi de fond 18 est réalisée sous la forme d'une pièce distincte, elle peut être assemblée à la paroi latérale périphérique 20 par tout moyen connu, par exemple par simple emboîtement serré, par collage, par soudage, par vissage par des clips de fixation mécanique, etc., et on pourra alors prévoir un joint d'étanchéité, pour assurer que, lorsque la paroi de fond 18 est assemblée à la paroi latérale périphérique 20, elle ferme de manière étanche l'espace interne fermé 12.

Pour la clarté de la description, on considère dans la suite de la description que l'axe central A1 est orienté verticalement et que la paroi de fond 18 est agencée à une extrémité inférieure du dispositif de contrôle microbiologique, la paroi latérale périphérique 20 s'étendant vers le haut, selon la direction de l'axe A1, depuis la paroi de fond 18. Cependant, les notions de verticalité, d'horizontalité, et les notions « haut », « bas », « supérieur » et « inférieur » ne sont utilisées que par référence à l'orientation du dispositif de contrôle microbiologique tel qu'illustré sur les figures, de manière relative entre elles, sans avoir de caractère limitatif sur la portée de l'invention ou sur l'orientation du dispositif de contrôle microbiologique en utilisation.

La paroi latérale périphérique 20 est par exemple une surface de révolution autour de l'axe central A1. Dans l'exemple illustré, la paroi latérale 20 est sensiblement cylindrique. Cependant, d'autres formes peuvent être proposées.

En conséquence, le couvercle 16 présente une paroi transversale 22, perpendiculaire à l'axe central A1, qui en l'occurrence présente sensiblement une forme circulaire correspondant à la géométrie d'un bord supérieur 24 de la paroi latérale périphérique 20 du corps principal 14. Dans l'exemple illustré, le couvercle 16 présente une collerette cylindrique 26, en l'occurrence cylindrique de révolution autour de l'axe central A1, qui s'étend vers le bas depuis une face inférieure de la paroi transversale 22 du couvercle. La collerette cylindrique 26 est destinée à venir s'engager selon la direction verticale de l'axe central A1, à l'intérieur d'une extrémité supérieure de la paroi latérale périphérique 20 du corps principal 14.

On remarque que, dans le premier exemple de réalisation, l'extrémité supérieure de la paroi latérale périphérique 20 du corps principal présente, sur une face interne, un décrochement transversal qui délimite une surface annulaire d'appui 28 d'axe central A1, tournée vers le haut. La collerette cylindrique 26 présente un bord inférieur 30 qui, lorsque le couvercle 16 est assemblé sur le corps principal 14, vient en regard de la surface annulaire d'appui 28.

Le dispositif de contrôle microbiologique 10 selon invention comporte un moyen de filtration microbiologique 32 qui est disposé dans l'espace interne fermé 12 et qui sépare, dans l'espace interne fermé, un premier compartiment 12a d'un second compartiment 12b de l'espace interne fermé 12.

Dans l'exemple illustré, le premier compartiment 12a est délimité au moins en partie par le couvercle 16, tandis que le second compartiment 12b est délimité au moins en partie par le corps principal 14. En effet, le moyen de filtration microbiologique 32 s'étend dans l'espace interne fermé 12 sensiblement transversalement, selon toute la section de l'espace interne fermé. Dans l'exemple, le moyen de filtration microbiologique 32 présente une forme sensiblement plane, ici la forme d'un disque. Il est de préférence agencé perpendiculairement à l'axe central A1.

Dans le premier exemple illustré, le moyen de filtration microbiologique 32 présente un bord périphérique 34 qui présente la même forme et les mêmes dimensions qu'une section de la face interne de la paroi périphérique latérale 20 du corps principal 14. Dans le premier exemple illustré, le bord périphérique 34 est destiné à venir en appui axialement vers le bas, directement ou indirectement, contre la surface annulaire d'appui 28 du corps principal 14. Comme on le verra plus loin, dans le premier exemple illustré, le bord périphérique 34 du moyen de filtration microbiologique 32 est de préférence prévu pour être serré axialement entre le bord inférieur 30 de la collerette cylindrique 26 du couvercle 16 et la surface annulaire d'appui 28 du corps principal 14.

Le dispositif de contrôle microbiologique comporte, à l'intérieur de l'espace interne fermé 12, une couche nutritive 36 imprégnée d'une composition d'un milieu de culture microbiologique, la couche nutritive 36 étant en contact avec le moyen de filtration microbiologique 32.

Dans les modes de réalisation illustrés, la couche nutritive 36 est un élément distinct du moyen de filtration microbiologique 32, tout en étant en contact avec le moyen de filtration microbiologique 32. La couche nutritive 36 et le moyen de filtration microbiologique 32 sont mis en contact l'un avec l'autre au sein du dispositif de contrôle microbiologique une fois celui-ci assemblé.

Dans ce cas, la couche nutritive 36 est située de préférence, avec les conventions exprimées plus haut, en-dessous du moyen de filtration microbiologique 32. Dans ce cas, la couche nutritive 36 est située dans le second compartiment 12b de l'espace interne fermé 12. Cependant, rien n'empêche de prévoir que la couche nutritive soit située, avec les conventions exprimées plus haut, au-dessus du moyen de filtration microbiologique 32. Dans ce cas particulier, la couche nutritive comprend avantageusement au moins un substrat chromogénique et/ou fluorogénique apte à permettre une détection directe ou indirecte d'une activité enzymatique ou métabolique des micro-organismes cibles. Le signal visuel généré par ledit au moins un substrat est alors visible au travers d'au moins une partie de l'épaisseur de la couche nutritive.

Dans les exemples illustrés, la couche nutritive 36 présente une forme sensiblement plane, ici la forme d'un disque. La couche nutritive 36 présente un bord périphérique 38 qui, de préférence, coïncide avec le bord périphérique 34 du moyen de filtration microbiologique 32. Ainsi, la couche nutritive 36 et le moyen de filtration 32 présentent la même forme. De la sorte, dans le premier exemple illustré, le bord périphérique 38 peut venir en appui axialement vers le bas contre la surface annulaire d'appui 28, en s'intercalant entre la surface annulaire d'appui 28 du corps principal 14 et le bord périphérique 34 du moyen de filtration microbiologique 32. Dans le premier exemple illustré, le bord périphérique 38 de la couche nutritive 36 est de préférence prévu pour être serré axialement, conjointement avec le bord périphérique 34 du moyen de filtration microbiologique 32, entre le bord inférieur 30 de la collerette cylindrique 26 du couvercle 16 et la surface annulaire d'appui 28 du corps principal 14.

Au sens de la présente invention, la couche nutritive 36 comprend un support contenant un milieu de culture microbiologique.

Le support peut être à base de divers composés absorbants, de préférence à très fort pouvoir de rétention d'eau, tels que la rayonne, le coton, les fibres cellulosiques naturelles ou modifiées chimiquement comme la carboxy-méthyl cellulose, les polymères chimiques absorbants ou superabsorbants tels que sels de polyacrylate, copolymère acrylate/acrylamide. Ce support peut être imprégné d'un milieu de culture microbiologique sous forme liquide. Ce milieu de culture microbiologique peut être avantageusement déshydraté, c'est à dire ayant une « Aw » (Activity of water, Activité en eau) incompatible avec le développement microbien. Alternativement, le support peut être recouvert ou imprégné à sec d'un milieu de culture microbiologique ou de ses constituants sous forme de poudre. Alternativement, l'imprégnation liquide peut, après déshydratation, être complétée par ajout de poudre.

On entend par milieu de culture microbiologique, un milieu comprenant des éléments nutritifs nécessaires à la survie et/ou à la croissance de microorganismes, notamment un ou plusieurs parmi les hydrates de carbone, dont les sucres, les peptones, les facteurs de croissances, les sels minéraux et/ou les vitamines, etc...

En pratique, l'homme du métier choisira le milieu de culture microbiologique en fonction des micro-organismes cibles, selon des critères parfaitement connus et à la portée de cet homme de l'art. La couche nutritive 36 peut contenir d'éventuels éléments additifs comme par exemple :

- un ou plusieurs agents sélectifs tels que des inhibiteurs ou des antibiotiques pour favoriser la croissance et le développement d'une espèce/souche de micro-organisme particulier plutôt qu'une autre ;
- des tampons, colorants.

D'une manière générale, la couche nutritive 36 peut en sus contenir un substrat permettant la détection d'une activité enzymatique ou métabolique des micro-organismes cibles grâce à un signal détectable directement ou indirectement. Pour une détection directe, ce substrat peut être lié à une partie faisant office de marqueur, fluorescent ou chromogène. Pour une détection indirecte, la couche nutritive selon l'invention peut comporter en sus un indicateur de pH, sensible à la variation de pH induite par la consommation du substrat et révélant la croissance des micro-organismes cibles. Ledit indicateur de pH peut être un chromophore ou un fluorophore. On citera comme exemples de chromophores le rouge neutre, le bleu d'aniline, le bleu de bromocresol. Les fluorophores comprennent par exemple la 4-méthylumbelliferone, les dérivés de l'hydroxycoumarine ou les dérivés de la résorufine. Ainsi, le substrat fluorescent de PC-PLC préférentiel lement utilisé pour la mise en oeuvre du procédé selon l'invention correspond au 4-Méthyl- Umbelliferyl-Choline Phosphate (4 MU-CP).

Selon un mode de réalisation préféré de l'invention, le milieu de culture microbiologique de la couche nutritive 36 est, dans une configuration de mise à disposition du dispositif de contrôle microbiologique avant utilisation, déshydraté. Dans ce cas, après imprégnation à sec du support de la couche nutritive par le milieu de culture microbiologique déshydraté, la couche nutritive 36 peut faire l'objet d'une opération de calandrage. Le calandrage, par la pression et la température de chauffe générées, permet la rétention et le maintien stable dans le temps du milieu de culture microbiologique déshydraté dans le support de la couche nutritive, en assurant la rétention des éléments nutritifs et des éventuels éléments additifs dans la couche nutritive. Le calandrage de la couche nutritive 36 permet également l'obtention d'une surface lisse et plane de la couche nutritive. Le calandrage permet en outre l'accélération de la réhydratation de la couche nutritive par rapport à une couche nutritive noncalandrée, du fait de la compression de la couche nutritive qu'il induit. Dans le cas où le support est formé de fibres, cette compression, associée à la présence du milieu déshydraté au sein de la couche nutritive 36, génère une forte augmentation du pouvoir capillaire de cette dernière, provoquant sa réhydratation quasi- instantanée. Cela peut aussi contribuer à un phénomène d'aspiration du moyen de filtration microbiologique 32 distinct disposé contre sa surface. Le moyen de filtration microbiologique 32 peut ainsi se retrouver plaqué contre la couche nutritive 36, assurant ainsi l'absence d'espace ou la réduction d'espace entre les deux, au profit d'une croissance et/ou survie microbienne optimale(s) sur toute la surface du moyen de filtration microbiologique 32. On peut ainsi éviter la présence de moyen de liaison (par exemple éviter la présence de couche liante) entre le moyen de filtration microbiologique 32 et la couche nutritive 36 quand sont ceux-ci sont distincts. Ceci représente un avantage significatif, dans la mesure où un tel moyen de liaison ralentirait le passage des éléments nutritifs et des éventuels éléments additifs de la couche nutritive 36 réhydratée vers les micro-organismes présents sur le moyen de filtration microbiologique 32, réduisant ainsi la croissance et/ou les chances de survie de ces micro-organismes.

Le moyen de filtration microbiologique 32 comporte un filtre qui est perméable à l'eau et qui retient les micro-organismes, notamment à sa surface. Dans les cas où le moyen de filtration microbiologique 32 est distinct de la couche nutritive 36, le moyen de filtration microbiologique 32 est perméable aux éléments nutritifs et aux éventuels éléments additifs compris dans la couche nutritive 36 située sous le moyen de filtration microbiologique 32. Ce filtre peut comprendre un corps poreux qui peut être constitué par un matériau qui par sa nature, sa taille, son arrangement stérique possède ces propriétés. Ce corps poreux peut avoir ces propriétés par l'aménagement de pores. Le moyen de filtration microbiologique 32 peut par exemple être à base de un ou plusieurs matériaux, ou dérivés de ces matériaux, parmi le latex, le polytetrafluoroethylene, le poly(vinylidene) fluoride, le polycarbonate, le polystyrène, le polyamide, le polysulphone, le polyethersulfone, la cellulose, un mélange de celluloses et nitrocellulose. Préférentiellement le moyen de filtration microbiologique 32 est réalisé sous la forme d'une membrane poreuse perméable aux éléments nutritifs et des éventuels éléments additifs compris dans la couche nutritive 36, et apte à retenir les micro-organismes à sa surface. De préférence, le moyen de filtration microbiologique 32 recouvre toute la couche nutritive 36. La Demanderesse a découvert que les membranes de microfiltration de l'eau (et d'une manière générale des liquides) actuellement commercialisées présentent généralement les propriétés requises pour être utilisées comme moyen de filtration microbiologique 32. Elles permettent d'obtenir une très bonne résistance au déchirement lors de la manipulation, une porosité maîtrisée, un surface lisse, une fine épaisseur et la plupart du temps une forte hydrophilie. Leur couleur, généralement blanche, permet d'optimiser la différentiation de colonies colorées sur leur surface. La capacité de filtration et l'hydrophilie d'une telle membrane de filtration, quant à elles, sont mises à profit pour permettre et optimiser le passage des éléments nutritifs et des éventuels éléments additifs présents dans la couche nutritive (éventuellement après sa réhydratation) vers une surface supérieure du moyen de filtration microbiologique 32 tout en empêchant ou limitant la migration en sens inverse des bactéries, levures etc.. filtrées à la surface supérieure du moyen de filtration microbiologique 32. Aux fins de la présente demande, les susdites membranes de filtration sont indifféremment désignées « membranes de filtration », « membranes de microfiltration » ou encore « membranes filtrantes », ces expressions étant synonymes les unes des autres. Ces membranes de filtration sont comprises dans le groupe constitué par les membranes poreuses.

Le moyen de filtration microbiologique 32 permet le passage des éléments du milieu nutritif et des agents sélectifs ou réactifs. Avantageusement, le moyen de filtration comporte des pores dont le diamètre est compris entre 0,01 et 0,8 microns préférentiellement entre 0,2 microns et 0,6 microns de manière à retenir les bactéries, levures et moisissures sur sa surface. Selon un mode de réalisation particulier, le moyen de filtration comporte des pores dont le diamètre est compris entre 0,25 microns et 0,6 microns, par exemple entre 0,3 microns et 0,6 microns, ou encore entre 0,4 microns et 0,6 microns. Alternativement, il peut s'agir d'une couche ne présentant pas de pores mesurables comme une membrane de dialyse.

Par exemple, un moyen de filtration microbiologique peut être une membrane de filtration « Fisherbrand™ General Filtration Membrane Filters » commercialisées par Fisher Scientific Company L.L.C, 300 Industry Drive, Pittsburgh, PA 15275, USA, ou encore une membrane de de filtration « Nitocellulose Membrane Filters », fabriquées par Zefon International, Inc., 5350 SW Ist Lane, Ocala, FL 34474, USA, ou des membranes analogues.

Dans certaines variantes, la couche nutritive peut être intégrée au moyen de filtration microbiologique 32, celui servant de support au milieu de culture microbiologique. Dans ce cas, il va de soi que la couche nutritive est en contact avec le moyen de filtration microbiologique 32.

Le dispositif de contrôle microbiologique 10 selon l'invention comporte un port d'entrée 40 pour le liquide à analyser. Le port d'entrée 40 permet, lorsque le dispositif de contrôle microbiologique 10 est assemblé de telle sorte que l'enceinte délimite l'espace interne fermé, par exemple lorsque le couvercle 16 est assemblé avec corps principal 14, d'introduire le liquide à analyser à l'intérieur de l'espace interne fermé délimité par l'enceinte, en provenance de l'extérieur de l'espace interne fermé 12.

Dans les exemples de réalisation illustrés, le port d'entrée 40 comprend une portion interne 42 qui débouche dans le premier compartiment 12a de l'espace interne fermé 12, et une portion externe 44 de raccordement à un contenant de liquide à analyser, le contenant pouvant être par exemple une seringue, une tubulure, une poche, un entonnoir etc.. Dans les exemples illustrés, le port d'entrée 40 est agencé selon l'axe central A1, verticalement. Le port d'entrée est avantageusement agencé sur le couvercle 16, en l'occurrence par exemple au centre de la paroi transversale 22 de celui-ci.

La portion interne 42 du port d'entrée 40 peut comporter un répartiteur 56 comportant plusieurs passages distincts pour le liquide à analyser. Un tel répartiteur 56 favorise la distribution du liquide à analyser, qui est introduit par le port d'entrée 40, sur une plus grande portion de la superficie du moyen de filtration microbiologique 32. Notamment dans le cas de la configuration de l'exemple de réalisation, la portion interne 42 du port d'entrée 40 peut comprendre un répartiteur 56 qui présente des orifices dont chacun débouche au moins en partie selon une direction radiale par rapport à l'axe central A1, les orifices étant de préférence répartis angulairement tout autour de l'axe central A1 du port d'entrée 40.

La portion externe 44 de raccordement du port d'entrée 40 peut comporter des moyens d'accouplement avec le contenant. La portion externe 44 de raccordement peut elle-même présenter une forme d'entonnoir. La portion externe 44 de raccordement peut comporter en plus des moyens d'arrimage mécanique pour arrimer le contenant au port d'entrée 40.

Le dispositif de contrôle microbiologique 10 comporte un obturateur 46 qui, dans les exemples de réalisation, est interposé entre la portion interne 42 et la portion externe 44 du port d'entrée, de manière à obturer le port d'entrée 40, empêchant, dans un état fermé de l'obturateur, toute circulation gazeuse entre l'espace interne fermé 12 du dispositif de contrôle microbiologique 10 et l'extérieur au travers du port d'entrée 40.

De préférence, le port d'entrée 40 est un port refermable. Dans ce cas, qui est celui illustré, l'obturateur 46 peut comporter une vanne. Une telle canne peut de préférence être amenée successivement plusieurs fois d'un état ouvert à un état fermé et inversement.

Dans certains cas, l'obturateur peut comporter une membrane étanche, et l'obturateur peut être est amené dans un état ouvert par rupture de la membrane. Dans l'hypothèse, la membrane ne peut pas être refermée. Dans ce cas, on peut prévoir de refermer le port d'entrée 40 par un obturateur secondaire rapporté sur la portion externe 44 de raccordement. Un tel obturateur secondaire (non représenté) peut être formé par exemple par un bouchon, une membrane étanche ou un capuchon.

On note qu'un tel obturateur secondaire peut aussi être prévu dans le cas de présence d'un obturateur de type vanne comme évoqué ci-dessus. Un tel obturateur secondaire permet par exemple de renforcer l'étanchéité aux gaz de la vanne, notamment à l'air, et notamment l'étanchéité à long terme pendant une période de stockage du dispositif 10 avant utilisation.

Dans les deux cas, un tel obturateur secondaire permet de protéger le port d'entrée de toute contamination pendant une période de stockage du dispositif 10 avant utilisation.

Dans les exemples illustrés, le dispositif de contrôle microbiologique 10, une fois assemblé, ne comporte qu'un seul port de communication fluidique entre l'espace interne fermé 12 et l'extérieur, ici le port d'entrée 40. Dans l'exemple illustré, on remarque que le second compartiment 12b de l'espace interne fermé 12 est dépourvu de port de communication fluidique avec l'extérieur de l'espace interne fermé. Cela n'empêche toutefois pas que le dispositif de contrôle microbiologique 10 pourrait comporter plusieurs ports de communication fluidique, dont le port d'entrée 40, débouchant tous dans le premier compartiment 12a de l'espace interne fermé.

Dans les exemples de réalisation, le moyen de filtration microbiologique 32 présente une faible épaisseur par rapport à son étendue. Par exemple, le diamètre du moyen de filtration est supérieur à 50 millimètres, par exemple compris entre 80 et 100 millimètres. Son épaisseur est de l'ordre de quelques millimètres, généralement inférieure à 5 millimètres.

Aussi, il est avantageux de prévoir que le dispositif de contrôle microbiologique 10 comporte un support 48 pour le moyen de filtration microbiologique 32 et pour la couche nutritive 36. Le support 48 permet de maintenir le moyen de filtration microbiologique 32 et la couche nutritive 36 dans leur position entre le premier compartiment 12a et le second compartiment 12b.

Dans le premier exemple de réalisation illustré, le support 48 pour le moyen de filtration microbiologique 32 et pour la couche nutritive 36 comprend des cloisons de support 50 agencées dans le second compartiment 12b.

Par exemple, les cloisons de support 50 peuvent être de forme plane, agencées chacune dans un plan radial contenant l'axe central A1. Elles peuvent par exemple s'étendre depuis la paroi de fond 18 du corps principal 14 et présenter un bord supérieur 52 contre lequel le moyen de filtration microbiologique 32 et la couche nutritive 36 peuvent venir en appui verticalement vers le bas, directement ou indirectement. Dans le premier exemple illustré, chaque cloison de support 50 s'étend diamétralement en travers de l'intégralité du second compartiment 12b, en étant donc limitée transversalement par deux portions diamétralement opposées de la paroi latérale périphérique 20 du corps principal 14. Dans ce mode de réalisation particulier, on comprend que les cloisons de support 50 exercent leur fonction de support par le biais de leur bord supérieur 52. Dans l'exemple illustré, les bords supérieurs 52 des cloisons de support 50 s'étendent tous dans un même plan transversal perpendiculaire à l'axe central A1.

Toutefois, en elles-mêmes, les cloisons de support 50 délimitent entre elles, dans le second compartiment 12b, des subdivisions de ce second compartiment 12b. De telles cloisons de support 50 peuvent être ajourées pour permettre la circulation de fluide de part et d'autre desdites cloisons dans le second compartiment 12b. Dans l'exemple illustré, il est choisi de munir les cloisons de support 50 d'ouverture traversantes 54 qui permettent une communication fluidique entre deux subdivisions adjacentes du second compartiment 12b qui sont séparées par une de ces cloisons de support 50. Ces ouvertures traversantes 54 sont optionnelles. Dans l'exemple illustré, elles sont réalisées sous la forme de fentes qui s'étendent selon la direction de l'axe central A1, depuis un point bas situé sensiblement à mi-hauteur du second compartiment 12b, et elles débouchent de manière ouverte dans le bord supérieur 52. Ces ouvertures traversantes pourraient présenter une tout autre géométrie et être par exemple réalisées sous la forme de trous, notamment circulaires. Elles ne sont pas nécessairement débouchantes dans le bord supérieur 52.

Dans l'exemple illustré, les cloisons de support 50 permettent aussi de renforcer mécaniquement l'enceinte du dispositif, notamment pour la rendre plus résistante à une différence de pression entre l'intérieur et l'extérieur de l'enceinte.

Le support 48 pour le moyen de filtration microbiologique 32 pourrait être réalisé de manière différente. Il pourrait par exemple être réalisé sous la forme d'une grille s'étendant dans un plan transversal perpendiculaire à l'axe central A1. Une telle grille pourrait par exemple être supportée en appui sur la surface d'appui 28 du corps principal 14. Le support pour le moyen de filtration microbiologique 32 pourrait être réalisé sous la forme d'une ou plusieurs colonnes s'étendant verticalement selon la direction de l'axe A1 depuis la paroi transversale de fond 18 du corps principal 14. Le support pour le moyen de filtration microbiologique 32 pourrait aussi être réalisé sous la forme d'une ou plusieurs consoles s'étendant transversalement depuis une face interne de la paroi latérale périphérique 20.

Dans le second exemple illustré aux Figs. 5 à 7, le support 48 pour le moyen de filtration comporte une grille 49. Une telle grille pourrait être formée par un faisceau de fils entrecroisés ou par un faisceau de barreaux entrecroisés. Dans l'exemple illustré aux Figs. 5 à 7, la grille est formée par une plaque perforée qui s'étend perpendiculairement à l'axe central A1, entre le premier compartiment et le second compartiment. Cette grille 49 est dans le cas illustré réalisée en une seule pièce avec la paroi latérale périphérique 20 du corps principal 14. On comprend que cette grille 49 en forme de plaque perforée assure un meilleur support pour le moyen de filtration microbiologique 32, surtout si celui-ci est peu rigide. Il en résulte une meilleure planéité du moyen de filtration microbiologique 32 et de la couche nutritive 36, notamment lors de la mise en oeuvre de procédé de filtration.

Dans l'exemple illustré, la plaque s'étend en travers du corps principal 14 sur tout le diamètre interne de celui-ci. La plaque présente une partie périphérique externe 51, annulaire, qui s'étend radialement vers l'axe central A1 depuis une surface cylindrique interne de la paroi latérale périphérique 20. La partie périphérique externe 51 de la plaque est pleine, donc non perforée. La plaque présente une partie centrale perforée, au centre de cette partie périphérique externe, qui forme la grille 49. La face supérieure de la partie centrale perforée 49 est décalée vers le bas par rapport à une surface supérieure de la partie périphérique externe. De la sorte, la partie périphérique externe 51, délimite, dans la face supérieure de la plaque, un renfoncement dont le diamètre correspond à celui de la partie centrale perforée 49. Dans l'exemple illustré, le moyen de filtration 32, et la couche nutritive 36, présentent un diamètre externe qui est égal ou inférieur au diamètre du renfoncement. Ainsi, le moyen de filtration 32, et la couche nutritive 36, peuvent être reçus dans le renfoncement, en étant calés radialement dans le renfoncement. On note, que, dans ce second exemple de réalisation, le moyen de filtration 32, et la couche nutritive 36, ne sont pas pincés entre le couvercle 16 et le corps principal 14, contrairement au premier exemple de réalisation.

On note sur la Fig.6, en vue de dessous, que le second exemple de réalisation comporte, dans le second compartiment 12b, des cloisons 53 qui, hormis la fonction de support direct de moyen de filtration, présentent les mêmes fonctions, et sensiblement la même géométrie que les cloisons de support du premier exemple de réalisation.

Le support 48 est dimensionné pour former une résistance limitée, voire négligeable à l'écoulement des fluides entre le premier et le second compartiment de l'espace interne fermé. Dans le cas d'une plaque perforée, on veillera par exemple à ce que la superficie totale cumulée, en projection selon l'axe central A1, des perforations 55 représente au moins 30% de la superficie du moyen de filtration 32, de préférence au moins 50% de la superficie du moyen de filtration. La plaque perforée présente une multitude de perforations 55, réparties à l'intérieur d'un cercle circonscrit (plus petit cercle contenant toutes les performations) qui est en correspondance avec au moins 50%, de préférence au moins 70 % de la superficie du moyen de filtration 32. Dans les exemples envisagés, les perforations 55 sont en nombre supérieur à 20, de préférence supérieur à 50. Cependant, avec des perforations plus grandes, et éventuellement de géométrie différente, par exemple en étoile, en éventail, etc., un nombre inférieur de perforations pourrait être utilisé.- Le support 48 pour le moyen de filtration microbiologique 32 est, dans l'exemple illustré, réalisé en une seule pièce avec le corps principal 14. Toutefois, ce support pourrait être réalisé sous la forme d'une ou plusieurs pièces indépendantes. De telles pièces peuvent être simplement posées à l'intérieur du second compartiment 12b, ou peuvent être assemblé au corps principal 14, par exemple par collage, par soudage, par encliquetage ou par emboîtement.

Dans l'exemple illustré, on note que la couche nutritive 36 est agencée entre le moyen de filtration microbiologique 32 et le support 48 pour le moyen de filtration.

Avantageusement, on peut prévoir que la couche nutritive 36 est serrée localement entre le moyen de filtration microbiologique 32 et son support 48. Par exemple, le couvercle 16 peut comporter des éléments d'appui, correspondant par exemple au bord supérieur d'une ou des cloisons de support 50, pour que, lorsque le dispositif de contrôle microbiologique est assemblé, le moyen de filtration microbiologique 32 et la couche nutritive 36 se retrouvent serrés entre ces éléments d'appui du couvercle 16 et le support 48. Dans l'exemple illustré, le répartiteur 56 présente, sur une face inférieure, de tels moyens d'appui pour, au centre du dispositif de contrôle microbiologique, serrer le moyen de filtration microbiologique 32 et la couche nutritive 36 contre le support 48.

Dans l'exemple illustré, le volume du second compartiment 12b de l'espace interne fermé 12 est supérieur au volume du premier compartiment 12a de l'espace interne fermé. De préférence, le volume du second compartiment 12b de l'espace interne fermé 12 est au moins deux fois, de préférence au moins trois fois supérieur au volume du premier compartiment 12a de l'espace interne fermé. Dans un mode de réalisation, pour un dispositif destinée à l'analyse d'un échantillon de 100 millilitres, le volume du second compartiment 12b de l'espace interne fermé 12 est par exemple d'au moins 100 millilitres, de préférence supérieur à 100 millilitres et inférieur à 150 millilitres, pour pouvoir contenir l'intégralité du volume d'un liquide à analyser. Le volume total de l'espace interne fermé délimité par l'enceinte est par exemple compris entre 120 et 300 millilitres, ce volume total étant par exemple de 150 millilitres.

Dans une configuration de mise à disposition du dispositif de contrôle microbiologique 10 avant utilisation, l'obturateur 46 est dans un état fermé pour fermer le port d'entrée 40 et l'espace interne fermé 12 de manière étanche à l'air.

Dans cette configuration de mise à disposition, le dispositif de contrôle microbiologique 10 est donc fermé de manière étanche, sans communication gazeuse possible entre l'espace interne fermé 12 délimité par l'enceinte et l'extérieur. Dans cette configuration de mise à disposition, le moyen de filtration microbiologique 32 et la couche nutritive 36 sont contenus à l'intérieur de cet espace interne fermé 12 délimité par l'enceinte, formant un ainsi un dispositif de contrôle microbiologique prêt à l'emploi pour filtrer le liquide à analyser afin d'en recueillir d'éventuels micro-organismes, sur le moyen de filtration microbiologique 32, et pour permettre le développement de ces micro-organismes en vue d'une détection, d'un dénombrement, d'une caractérisation et/ou une identification.

De plus, dans cette configuration de mise à disposition du dispositif de contrôle microbiologique 10 avant l'utilisation, la pression gazeuse absolue à l'intérieur de l'espace interne fermé est à une valeur de pression initiale réduite de telle sorte que le dispositif est apte à créer une aspiration au travers du port d'entrée lors d'une première ouverture de l'obturateur 46. Il résulte des deux paragraphes précédents que, dans cette configuration de mise à disposition du dispositif de contrôle microbiologique 10 avant l'utilisation, donc avant toute introduction d'un échantillon dans cet espace interne fermé 12 délimité par l'enceinte, le moyen de filtration microbiologique 32 et la couche nutritive 36, laquelle comprend une composition d'un milieu de culture microbiologique, sont contenus à l'intérieur de cet espace interne fermé 12 délimité par l'enceinte et la pression gazeuse absolue à l'intérieur de l'espace interne fermé est à une valeur de pression initiale réduite de telle sorte que le dispositif est apte à créer une aspiration au travers du port d'entrée lors d'une première ouverture de l'obturateur 46.

Pour cela, la valeur de pression initiale réduite de la pression gazeuse absolue à l'intérieur de l'espace interne fermé (12), ramenée à une température de 25°C, est strictement inférieure à la pression atmosphérique standard de 100 kPa à 25°C.

En pratique, ce phénomène d'aspiration se traduira par une entrée, dans l'espace interne du dispositif, d'un volume prédéterminé de liquide de manière plus rapide, lors de la première ouverture de l'obturateur, que dans le cas où la pression initiale dans l'espace interne serait égale à la pression atmosphérique.

On notera qu'il n'est pas nécessaire de connaître avec précision la valeur précise de la pression initiale réduite. En effet, cette valeur est avant tout déterminée de manière à être suffisante pour aspirer au moins en partie, voire en totalité, un volume prédéterminé de l'échantillon à analyser à l'intérieur du dispositif.

De préférence, cette valeur est déterminée de manière à être suffisante pour que le dispositif soit apte à aspirer en totalité un volume prédéterminé de l'échantillon à analyser à l'intérieur du dispositif, sans qu'il soit nécessaire de soumettre l'échantillon à analyser à une pression supérieure à la pression atmosphérique standard pour le faire entrer à l'intérieur du dispositif.

De préférence, cette valeur de pression initiale réduite est suffisamment basse pour permettre l'entrée en totalité d'un volume prédéterminé de l'échantillon à analyser dans l'espace interne sans évacuation de fluide depuis l'espace interne lors de l'entrée du volume prédéterminé de l'échantillon à analyser. Cela permet de s'assurer d'une entrée facile de tout l'échantillon dans le dispositif. Cela permet aussi d'éviter toute propagation d'éléments initialement contenus dans le dispositif, notamment d'éléments du milieu de culture, vers l'extérieur, lors de l'entrée de l'échantillon dans le dispositif.

L'homme du métier pourra, par une évaluation initiale, éventuellement complétée de quelques essais, déterminer une pression initiale réduite souhaitée pour le dispositif, en fonction des conditions envisagées pour la mise en oeuvre du dispositif (volume total de l'espace interne fermé 12 délimité par l'enceinte du dispositif, volume de l'échantillon, conditions de température et de pression lors de la mise en oeuvre du dispositif, ...).

De manière pratique, on peut évaluer la valeur de la pression initiale réduite souhaitée de la manière suivante. On considère le volume total VT de l'espace interne fermé 12 délimité par l'enceinte. On prédétermine ensuite un volume prédéterminé VE de l'échantillon à analyser que l'on souhaite pouvoir introduire dans le dispositif 10 pour effectuer l'analyse. On en déduit ensuite le volume libre final VL dans l'espace interne, après l'entrée du volume prédéterminé de l'échantillon à analyser dans l'espace interne. Ce volume libre final VL vaut donc le volume total VT de l'espace interne fermé 12 délimité par l'enceinte auquel on soustrait le volume prédéterminé VE de l'échantillon à analyser que l'on souhaite pouvoir introduire dans le dispositif 10 pour effectuer l'analyse : VL = VT - VE.

On applique ensuite, à titre d'approximation, la loi des gaz parfaits aux variations de conditions dans l'espace interne fermé 12 délimité par l'enceinte entre l'instant juste avant l'entrée de l'échantillon et l'instant juste après l'entrée de cet échantillon, en supposant que seulement du liquide contenant l'échantillon est introduit, sans variation de température significative. Cette entrée de l'échantillon se traduit par une variation de la pression depuis la valeur de pression initiale réduite Pi, qui est la valeur de la pression gazeuse absolue à l'intérieur de l'espace interne fermé dans la configuration de mise à disposition, jusqu'à une valeur finale Pf, qui est la valeur pression gazeuse absolue à l'intérieur de l'espace interne, après l'entrée d'un volume prédéterminé de l'échantillon à analyser.

On obtient donc la relation

$$Pf \times VL = Pf \times (VT - VE) = Pi \times VT$$

ce qui donne

$$Pi = Pf \times (VT - VE) / VT = Pf \times VL / VT$$

On en déduit donc que dans la configuration de mise à disposition du dispositif de contrôle microbiologique 10 avant utilisation, la pression gazeuse absolue à l'intérieur de l'espace interne fermé 12, dite pression initiale réduite, est de préférence strictement inférieure à la pression atmosphérique multipliée par le rapport du volume libre final dans l'espace interne, après l'entrée d'un volume prédéterminé de l'échantillon à analyser, divisé par le volume total de l'espace interne.

De manière pratique, on peut fixer de manière arbitraire la valeur de la pression atmosphérique standard à 100 kPa à 25°C.

Dans la pratique, on considère généralement qu'un échantillon doit avoir un volume VE d'au moins 20 millilitres, de préférence au moins 50 millilitres, plus préférentiellement au moins 100 millilitres. En revanche on considère généralement qu'un échantillon doit avoir un volume VE de 300 millilitres ou moins, de préférence 200 millilitres ou moins, plus préférentiellement 150 millilitres ou moins.

Dans le cas d'un dispositif dont le volume total de l'espace interne VT est de 150 millilitres, et pour un échantillon de volume VE 100 millilitres, le rapide calcule ci-dessus donne la pression initiale réduite souhaitée, pour la pression gazeuse absolue à l'intérieur de l'espace interne fermé 12 dans la configuration de mise à disposition du dispositif avant utilisation, strictement inférieure à 33,3 kPa absolus. Cependant, en pratique, on préférera prévoir une pression initiale réduite strictement inférieure à 30 kPa absolus, pour tenir compte des approximations liées au décalage entre les hypothèses formulées et la réalité expérimentale. Plus préférentiellement, on préférera prévoir une pression initiale strictement inférieure à 20 kPa absolus, notamment pour favoriser une entrée rapide de l'échantillon dans le dispositif.

On notera que, pour un volume total VT de 300 millilitres de l'espace interne du dispositif, on détermine ainsi une pression initiale réduite souhaitée strictement inférieure à 66,6 kPa absolus, de préférence strictement inférieure à 60 kPa absolus, plus préférentiellement strictement inférieure à 40 kPa absolus.

On note que les valeurs ci-dessus sont des valeurs indicatives, y compris pour un dispositif donné et des conditions de mise en oeuvre données. On aura en effet, tout intérêt à ce que la pression initiale réduite dans le dispositif soit effectivement inférieure aux valeurs ci-dessus.

Dans la pratique, ces valeurs pourront servir de base pour la mise au point d'un dispositif selon l'invention, et que les conditions de fabrication permettant le bon fonctionnement du dispositif seront aisément déterminées avec quelques essais de routine.

Aussi, les valeurs ci-dessus pourront être mesurées en branchant un manomètre à l'entrée du port d'entrée, au plus près de l'obturateur 46, même s'il en résulte une incertitude sur la valeur réelle de la pression initiale réduite, y compris si cette incertitude atteint 5 kPa.

Cela signifie que, au cours de la préparation du dispositif de contrôle microbiologique, un vide au moins partiel est créé dans l'espace interne fermé. Ce vide au moins partiel peut avoir été par exemple réalisé par un assemblage sous vide, ou tout au moins sous une pression inférieure ou égale à la pression initiale réduite souhaitée, en tous cas strictement inférieure à 100 kPa à 25°C, ou par dépressurisation de l'espace interne fermé après assemblage du dispositif de contrôle microbiologique.

On comprend que, dans cette configuration de mise à disposition du dispositif de contrôle microbiologique 10 avant l'utilisation, l'espace interne fermé est isolé de toute source externe d'aspiration. On comprend donc la nécessité de prévoir que l'enceinte et le port d'entrée 40 dans son état fermé soient étanches aux gaz, notamment étanches à l'air. Cela est obtenu par tout moyen connu de l'homme du métier. Dans l'exemple illustré, le couvercle 16 et le corps principal 14 sont ainsi assemblés de manière étanche à l'air.

L'assemblage peut être un assemblage démontable, permettant une ouverture du dispositif de contrôle microbiologique sans destruction après son utilisation, par exemple pour en retirer le moyen de filtrage microbiologique 32. Un assemblage démontable peut être réalisé par exemple au moyen de filetages complémentaires agencés respectivement sur le couvercle 16 et le corps principal 16. Dans la configuration illustrée, de tels filetages complémentaires (non représentés) peuvent être aménagés respectivement sur une face externe de la collerette cylindrique 26 du couvercle 16 et sur une face interne de l'extrémité supérieure de la paroi latérale périphérique 20 du corps principal 14. Un autre exemple possible d'assemblage démontable peut être obtenu par un système d'assemblage à baïonnette. Encore un autre exemple d'assemblage démontable peut être obtenu en prévoyant une bride externe d'assemblage, ou en prévoyant des vis d'assemblage du couvercle 16 sur le corps principal 14.

L'assemblage peut être un assemblage non-démontable, ne permettant pas une ouverture du dispositif de contrôle microbiologique 10 sans destruction après son utilisation, par exemple par collage, par soudage ou par rivetage.

Pour assurer l'étanchéité requise aux gaz, notamment à l'air, on peut prévoir, notamment dans le cas d'un assemblage démontable, un ou plusieurs joints d'étanchéité (non représentés) entre le corps principal 14 et le couvercle 16.

Dans la configuration de mise à disposition du dispositif de contrôle microbiologique avant utilisation, le milieu de culture microbiologique de la couche nutritive 36 est avantageusement déshydraté. Il est alors prévu que ce milieu de culture microbiologique soit réhydraté au moment de l'utilisation. Cette réhydratation peut être réalisée par le liquide à analyser lui-même.

En effet, le moyen de filtration microbiologique 32 et la couche nutritive 36 sont agencés pour que tout échange fluidique entre le premier compartiment 12a et le second compartiment 12b de l'espace interne fermé se fasse au travers du moyen de filtration microbiologique 32 et de la couche nutritive 36. On peut ainsi prévoir qu'il ne soit pas possible pour un fluide de contourner ni le moyen de filtration microbiologique 32 ni la couche nutritive 36 pour passer du premier compartiment 12a au second compartiment 12b. Dans le mode de réalisation illustré, cela découle du fait que le moyen de filtration microbiologique 32 et la couche nutritive 36 s'étendent en travers de l'intégralité de la section de l'espace interne fermé 12 du dispositif de contrôle microbiologique entre le premier compartiment 12a et le second compartiment 12b de l'espace interne fermé.

Avantageusement, il peut être prévu qu'un matériau absorbant l'eau soit agencé dans le second compartiment. Dans l'exemple illustré, un tel matériau peut être agencé dans une, plusieurs, ou toutes parmi les subdivisions du second compartiment 12b entre les cloisons de support 50. Un matériau absorbant peut être à base de divers composés absorbants, de préférence à très fort pouvoir de rétention d'eau, tels que la rayonne, le coton, les fibres cellulosiques naturelles ou modifiées chimiquement comme la carboxy-méthyl cellulose, les polymères chimiques absorbants ou superabsorbants tels que sels de polyacrylate, copolymère acrylate/acrylamide. Des tels matériaux peuvent ainsi être obtenus auprès de la société Technical Absorbents Limited, 1 Moody Lane, Great Coates, Grimsby, DN31 2SS, Royaume-Uni, sous la marque « Super Absorbent Fibre (SAF®) ».

L'enceinte du dispositif de contrôle microbiologique peut être avantageusement réalisée en matériau polymère. Cependant, il est aussi possible de la réaliser en d'autres matériaux, y compris au moins en partie en verre. Dans l'exemple illustré, le corps principal 14, le couvercle 16 et le support 48 peuvent être réalisés dans le même matériau, ou en des matériaux différents.

De préférence, l'enceinte du dispositif de contrôle microbiologique comporte au moins une partie transparente. Notamment, cette partie transparente peut être aménagée de manière à ce qu'un observateur puisse voir au moins une partie de la face supérieure du moyen de filtration microbiologique 32 qui est tournée vers le premier compartiment 12a. De préférence, cette partie transparente est aménagée de manière à ce qu'un observateur puisse voir l'intégralité de la face supérieure du moyen de filtration microbiologique 32 qui est tournée vers le premier compartiment 12a. En effet, c'est sur cette face que seront visibles d'éventuels micro-organismes, après incubation. Dans l'exemple illustré, la partie transparente de l'enceinte est donc de préférence aménagée au moins dans la paroi transversale 22 du couvercle 16. L'intégralité du couvercle 16 peut être transparente. Dans certains modes de réalisation, on prévoira que l'intégralité de l'enceinte est en matériau transparent. La partie transparente de l'enceinte est par exemple réalisée en poly(méthacrylate de méthyle) (PMMA) ou en verre.

Un dispositif de contrôle microbiologique tel que décrit ci-dessus a donc vocation à être utilisé dans un procédé de contrôle d'un liquide à analyser susceptible de contenir au moins un micro-organisme.

Dans une telle utilisation, on fournit préalablement un dispositif de contrôle microbiologique dans une configuration de mise à disposition avant utilisation. Comme on l'a vu plus haut, dans cette configuration, le dispositif de contrôle microbiologique dispose du moyen de filtration microbiologique 32 et de la couche nutritive 36 qui sont enfermés dans l'espace interne fermé 12 de manière étanche, et, dans cet espace interne fermé, il règne un niveau de dépression qui correspond à la pression initiale réduite, en tous cas à une pression gazeuse absolue à l'intérieur de l'espace interne fermé, ramenée à une température de 25°C, strictement inférieure à 100 kPa.

Pour mettre à disposition un dispositif de contrôle microbiologique pour contrôler un liquide à analyser susceptible de contenir au moins un micro-organisme, on doit donc d'abord fournir un dispositif de contrôle microbiologique comportant, comme décrit ci-dessus : -

une enceinte prévue pour délimiter un espace interne fermé 12 fermé destiné à recevoir le liquide à analyser, par exemple sous la forme d'un corps principal 14 et d'un couvercle 16 ; - un moyen de filtration microbiologique 32 prévu pour être disposé dans l'espace interne fermé 12 et pour séparer, dans l'espace interne fermé, un premier compartiment 12a d'un second compartiment 12b de l'espace interne fermé ;- un port d'entrée 40 pour le liquide à analyser prévu pour déboucher dans le premier compartiment 12a de l'espace interne fermé, le port d'entrée pouvant par exemple comprendre une portion interne 42 prévue pour déboucher dans le premier compartiment 12a de l'espace interne fermé, et une portion externe 44 de raccordement.

Par ailleurs, le procédé de mise à disposition, avant utilisation, comprend la fourniture d'une couche nutritive 36, prévue pour être reçue à l'intérieur de l'espace interne fermé et comprenant une composition d'un milieu de culture microbiologique, la couche nutritive 36 étant destinée à être en contact avec le moyen de filtration. Comme vu précédemment, cette couche nutritive 36 peut être distincte du moyen de filtration microbiologique 32 ou, en variante, on peut prévoir que la couche nutritive et le moyen de filtration microbiologique soient intégrés l'un à l'autre.

Selon l'invention, le procédé de mise à disposition du dispositif de contrôle microbiologique dans une configuration de mise à disposition comprend, avant toute utilisation donc avant tout raccordement à un contenant de liquide à analyser, successivement et dans cet ordre : - une étape de dépressurisation pour abaisser la pression gazeuse absolue à l'intérieur de l'espace interne fermé 12 ; - une étape d'obturation pour fermer l'espace interne fermé 12 de manière étanche à l'air.

On note qu'au moment de l'étape de dépressurisation, le dispositif de contrôle microbiologique 10 comprenant les éléments énoncés ci-dessus est déjà assemblé, de telle sorte que l'enceinte soit fermée et renferme les éléments ci-dessus. Dans ce cas, l'étape de dépressurisation peut être réalisée en reliant l'espace interne fermé 12 du dispositif de contrôle microbiologique à une source d'aspiration, par exemple une pompe à vide, par exemple au travers du port d'entrée 40, celui-ci étant alors dans un état ouvert. De ce fait, on abaisse la pression jusqu'à la pression initiale réduite souhaitée qui est, en tous cas, ramenée à une température de 25°C, strictement inférieure à 100 kPa

Dans une autre variante non revendiquée, l'étape de dépressurisation peut être concomitante à une étape d'assemblage. En effet, dans l'exemple illustré on peut prévoir par exemple que l'assemblage du couvercle 16 sur le corps principal 14, qui permet de fermer l'enceinte et donc de délimiter l'espace interne fermé 12, peut se faire sous une pression gazeuse absolue égale ou inférieure à la pression initiale réduite souhaitée, soit notamment une pression, ramenée à une température de 25°C, strictement inférieure à 100 kPa.

Dans le premier cas, l'étape d'obturation peut consister en la fermeture d'une vanne du port d'entrée 40 ou la pose d'une membrane d'étanchéité, tant que l'on est encore sous une pression gazeuse absolue égale ou inférieure à la pression initiale réduite souhaitée. Dans le second cas, l'étape d'obturation peut consister en l'assemblage du couvercle 16 sur le corps principal 14 de manière étanche qui permet de fermer l'enceinte. Dans ce cas, l'obturateur du port d'entrée 40 est de préférence préalablement dans un état fermé.

Ainsi, on obtient un dispositif de contrôle microbiologique 10 dans une configuration de mise à disposition dans laquelle, à l'intérieur de l'espace interne fermé 12 délimité par l'enceinte, on trouve le moyen de filtration microbiologique 32 et la couche nutritive 36, l'espace interne fermé 12 étant à un niveau initial de dépression prédéterminé, appelé pression initiale réduite, et qui correspond à une pression gazeuse dans l'espace interne fermé inférieure à un seuil prédéterminé, le seuil prédéterminé étant lui-même strictement inférieur à la pression atmosphérique standard, le seuil prédéterminé étant par exemple par exemple de 20 kPa de pression absolue ramenée à 25 °C. On note que, dans cette configuration de mise à disposition, le dispositif de contrôle microbiologique peut être stocké, transporté, etc...., et que dans cette configuration de mise à disposition, aucun liquide à analyser n'a été introduit dans l'espace interne fermé 12 du dispositif de contrôle microbiologique.

L'utilisation d'un dispositif de contrôle microbiologique selon l'invention correspond à l'introduction, à l'intérieur de l'espace interne fermé 12, du liquide à analyser, par le port d'entrée 40. Cette introduction correspond généralement au raccordement d'un contenant, dans lequel se trouve le liquide à analyser, avec le port d'entrée 40. Un tel raccordement peut prendre des formes variées, supposant simplement qu'une communication fluidique soit établie entre le contenant et le port d'entrée 40. De préférence, ce raccordement est un raccordement étanche aux fluides, et préférentiellement étanche aux gaz, notamment à l'air. Ce raccordement peut comprendre un arrimage mécanique entre le contenant et le port d'entrée 40.

De la sorte, l'utilisation d'un dispositif de contrôle microbiologique selon l'invention comporte les étapes consistant à : - raccorder un contenant de liquide à analyser au port d'entrée 40, notamment dans l'exemple de réalisation à la portion externe 42 de raccordement du port d'entrée 40; - ouvrir l'obturateur 46 du port d'entrée pour permettre le passage du liquide à analyser du contenant vers l'espace interne fermé 12 du dispositif de contrôle microbiologique.

Cette étape ouvrir l'obturateur 46 du port d'entrée est une première ouverture de l'obturateur suivant une étape d'obturation pour fermer l'espace interne fermé 12 de manière étanche lors du procédé de mise à disposition.

C'est à cette étape que le niveau initial de dépression prédéterminé joue un rôle particulièrement important. En effet, la présence de cette dépression est favorable à l'introduction du liquide à analyser dans l'espace interne fermé 12 du dispositif de contrôle microbiologique. Cela est dû d'une part au phénomène d'aspiration exercé sur le liquide à analyser si celui-ci est initialement par exemple à la pression atmosphérique. Cela est dû d'autre part au fait que le niveau de dépression prédéterminé se traduit par la présence en faible quantité, de gaz dans le dispositif de contrôle microbiologique avant l'introduction du liquide à analyser, de sorte que le dispositif de contrôle microbiologique ne doit pas, lors de l'introduction du liquide à analyser, évacuer une quantité correspondante de gaz. Non seulement cela facilite l'entrée du liquide analyser dans l'espace interne fermé du dispositif de contrôle microbiologique, mais cela évite l'expulsion vers l'extérieur de particules ou molécules initialement contenues dans le dispositif de contrôle microbiologique vers l'extérieur.

On note que pendant cette étape d'utilisation du dispositif de contrôle microbiologique au cours de laquelle l'ouverture de l'obturateur permet le passage du liquide à analyser du contenant vers l'espace interne fermé 12, l'espace interne fermé 12 peut être isolé de toute source externe d'aspiration. En effet, l'aspiration est avantageusement obtenue grâce à la pression initiale réduite présente dans le dispositif avant la première ouverture du dispositif suivant une étape d'obturation pour fermer l'espace interne fermé 12 de manière étanche à l'air lors du procédé de mise à disposition.

De la sorte, l'introduction du liquide à analyser dans le dispositif de contrôle microbiologique 10 selon l'invention, au travers du port d'entrée 40, permet au liquide d'être introduit tout d'abord dans le premier compartiment 12a de l'espace interne fermé 12, le liquide à analyser venant alors naturellement au contact du moyen de filtration microbiologique 32. Le liquide à analyser est donc filtré au travers de ce moyen de filtration microbiologique 32, de sorte que certains au moins des éventuels microorganismes, notamment ceux ciblés pour le contrôle envisagé, sont retenus par le moyen de filtration microbiologique 32. Au contraire, la partie liquide du liquide à analyser migre en direction du second compartiment 12b de l'espace interne fermé 12. Pour cela, on comprend qu'il est avantageux que le dispositif de contrôle microbiologique soit, pour cette étape au moins, dans l'orientation illustrée sur les figures, le second compartiment 12b de l'espace interne fermé 12 se trouvant en dessous du premier compartiment, les deux compartiments étant séparés l'un de l'autre par le moyen de filtration microbiologique 32 qui, dans l'exemple illustré, s'étend selon un plan qui est alors horizontal.

Le liquide à analyser permet la réhydratation de la couche nutritive 36.

Comme la couche nutritive 36 est au contact du moyen de filtration, les éléments nutritifs et les éventuels éléments additifs de celle-ci peuvent migrer en direction des micro-organismes qui sont retenus par le moyen de filtration microbiologique 32. De la sorte, pour peu que le dispositif de contrôle microbiologique 10 soit mis dans un environnement, notamment de température, favorable, on peut obtenir une incubation des microorganismes à l'intérieur du dispositif de contrôle microbiologique 10 lui-même, sans qu'il soit nécessaire d'ouvrir celui-ci, en tout cas sans qu'il soit nécessaire de retirer le moyen de filtration microbiologique 32 de l'enceinte du dispositif de contrôle microbiologique 10.

Ainsi, l'utilisation d'un dispositif de contrôle microbiologique 10 selon l'invention peut, après l'étape d'introduction d'un liquide à analyser à l'intérieur de l'espace interne fermé 12 du dispositif de contrôle microbiologique, comprendre les étapes ultérieures consistant à :

- refermer l'obturateur 46 du port d'entrée 40 ;
- faire incuber, dans le dispositif de contrôle microbiologique, un éventuel micro-organisme contenu initialement dans le liquide à analyser.

Après une telle période d'incubation, l'utilisation peut comporter une étape ultérieure de contrôle consistant à détecter, dénombrer, identifier et/ou caractériser visuellement un éventuel micro-organisme contenu initialement dans le liquide à analyser, notamment par vision au travers d'une portion transparente de l'enceinte du dispositif de contrôle microbiologique. Là encore, cette étape de contrôle peut être réalisée sans qu'il soit nécessaire d'ouvrir le dispositif de contrôle microbiologique 10, le moyen de filtration microbiologique 32, sur lequel se trouvent les éventuels micro-organismes, restant donc à l'intérieur de l'espace interne fermé du dispositif de contrôle microbiologique 10. L'invention n'est pas limitée aux exemples décrits et représentés car diverses modifications peuvent y être apportées sans sortir de son cadre.

## Revendications

1. Dispositif de contrôle microbiologique (10) pour contrôler un liquide à analyser susceptible de contenir au moins un micro-organisme, dans une configuration de mise à disposition du dispositif de contrôle microbiologique (10) avant utilisation et comportant:

   - un espace interne fermé (12) délimité par une enceinte et destiné à recevoir le liquide à analyser ;
   - un moyen de filtration microbiologique (32) disposé dans l'espace interne fermé (12) et séparant, dans l'espace interne fermé, un premier compartiment (12a) d'un second compartiment (12b) de l'espace interne fermé ;
   - un port d'entrée (40) pour le liquide à analyser, le port d'entrée débouchant dans le premier compartiment (12a) de l'espace interne fermé,

   **caractérisé en ce que** le dispositif de contrôle microbiologique (10) comporte, à l'intérieur de l'espace interne fermé, une couche nutritive (36) comprenant une composition d'un milieu de culture microbiologique, la couche nutritive (32) étant en contact avec le moyen de filtration (32),

en ce que le port d'entrée (40) du dispositif de contrôle microbiologique comporte un obturateur (46),
et en ce que :

- l'obturateur (46) du port d'entrée (40) est dans un état fermé pour fermer le port d'entrée (40) et l'espace interne fermé (12) de manière étanche à l'air ;
- la pression gazeuse absolue à l'intérieur de l'espace interne fermé (12), ramenée à une température de 25°C, est strictement inférieure à la pression atmosphérique standard de 100 kPa à 25°C, de manière que le dispositif est apte à créer une aspiration au travers du port d'entrée lors d'une première ouverture de l'obturateur (46),
- l'espace interne fermé (12) étant isolé de toute source externe d'aspiration.

2. Dispositif de contrôle microbiologique selon la revendication précédente, dans lequel le milieu de culture microbiologique de la couche nutritive (36) est déshydraté.

3. Dispositif de contrôle microbiologique selon l'une quelconque des revendications précédentes, **caractérisé en ce que** le dispositif de contrôle microbiologique (10) comporte un support (48) pour le moyen de filtration (32) et la couche nutritive (36).

4. Dispositif de contrôle microbiologique selon la revendication 3, **caractérisé en ce que** la couche nutritive (36) est serrée localement entre le moyen de filtration (32) et le support (48) pour le moyen de filtration (32).

5. Dispositif de contrôle microbiologique selon l'une quelconque des revendications 3 ou 4, **caractérisé en ce que** le support (48) pour le moyen de filtration (32) comprend des cloisons de support (50) agencées dans le second compartiment (12b).

6. Dispositif de contrôle microbiologique selon l'une quelconque des revendications 3 ou 4, **caractérisé en ce que** le support (48) pour le moyen de filtration (32) comprend une grille (49) qui s'étend en travers de l'espace interne fermé, entre le premier compartiment (12a) et le second compartiment (12b).

7. Dispositif de contrôle microbiologique selon l'une quelconque des revendications précédentes, **caractérisé en ce qu'**un matériau absorbant l'eau est agencé dans le second compartiment (12b).

8. Dispositif de contrôle microbiologique selon l'une quelconque des revendications précédentes, **caractérisé en ce que** l'obturateur (46) du port d'entrée (40) comporte une vanne.

9. Dispositif de contrôle microbiologique selon l'une quelconque des revendications précédentes, **caractérisé en ce que** l'enceinte du dispositif de contrôle microbiologique comporte au moins un corps principal (14) qui délimite au moins en partie le second compartiment (12b), et comporte un couvercle (16) qui délimite au moins en partie le premier compartiment (12a), le corps principal et le couvercle étant formés de pièces distinctes assemblées l'une à l'autre pour former le dispositif de contrôle microbiologique.

10. Dispositif de contrôle microbiologique selon l'une quelconque des revendications précédentes, **caractérisé en ce que** l'enceinte du dispositif de contrôle microbiologique comporte au moins une partie transparente.

11. Dispositif de contrôle microbiologique selon l'une quelconque des revendications précédentes, **caractérisé en ce que** le port d'entrée (40) comporte un répartiteur (46) comportant plusieurs passages distincts pour le liquide à analyser.

12. Dispositif de contrôle microbiologique selon l'une quelconque des revendications précédentes, **caractérisé en ce que**, dans la configuration de mise à disposition du dispositif de contrôle microbiologique (10) avant utilisation, la pression gazeuse absolue à l'intérieur de l'espace interne fermé (12) est telle qu'elle permet l'entrée d'un volume prédéterminé de l'échantillon à analyser sans évacuation de fluide depuis l'espace interne lors de l'entrée de l'entrée d'un volume prédéterminé de l'échantillon à analyser.

13. Dispositif de contrôle microbiologique selon l'une quelconque des revendications précédentes, **caractérisé en ce que**, dans la configuration de mise à disposition du dispositif de contrôle microbiologique (10) avant utilisation, la pression gazeuse absolue à l'intérieur de l'espace interne fermé (12) est strictement inférieure à la pression atmosphérique standard multipliée par le rapport du volume libre final dans l'espace interne, après l'entrée d'un volume prédéterminé de l'échantillon à analyser, divisé par le volume total de l'espace interne.

14. Dispositif de contrôle microbiologique selon l'une quelconque des revendications précédentes, **caractérisé en ce que**, dans la configuration de mise à disposition du dispositif de contrôle microbiologique

(10) avant utilisation, la pression gazeuse absolue à l'intérieur de l'espace interne fermé (12), ramenée à une température de 25°C, est strictement inférieure à 60 kPa absolus, de préférence strictement inférieure à 30 kPa absolus, plus préférentiellement strictement inférieure à 20 kPa absolus.

15. Procédé de mise à disposition d'un dispositif de contrôle microbiologique selon l'une des revendications précédentes et configuré pour contrôler un liquide à analyser susceptible de contenir au moins un microorganisme, comprenant la fourniture d'un dispositif de contrôle microbiologique (10) **caractérisé en ce que** le procédé comprend la fourniture d'une couche nutritive (32)reçue à l'intérieur de l'espace interne fermé (12) et comprenant une composition d'un milieu de culture microbiologique, la couche nutritive (36) étant en contact avec le moyen de filtration (32), et **en ce que** le procédé comprend, avant tout raccordement du dispositif de contrôle microbiologique (10) à un contenant de liquide à analyser, successivement et dans cet ordre :

- une étape de dépressurisation pour abaisser la pression gazeuse absolue à l'intérieur de l'espace interne fermé (12), ramenée à une température de 25°C, strictement inférieure à 100 kPa ;
- une étape d'obturation pour fermer l'espace interne fermé (12) de manière étanche à l'air.

16. Procédé selon la revendication 15, **caractérisé en ce que**, l'étape de dépressurisation abaisse la pression gazeuse absolue à l'intérieur de l'espace interne fermé (12) à une valeur telle qu'elle permet l'entrée d'un volume prédéterminé de l'échantillon à analyser sans évacuation de fluide depuis l'espace interne lors de l'entrée de l'entrée d'un volume prédéterminé de l'échantillon à analyser.

17. Procédé de contrôle microbiologique selon l'une quelconque des revendications 15 ou 16, **caractérisé en ce que** l'étape de dépressurisation abaisse la pression gazeuse absolue à l'intérieur de l'espace interne fermé (12) à une valeur strictement inférieure à la pression atmosphérique standard multipliée par le rapport du volume libre final dans l'espace interne, après l'entrée d'un volume prédéterminé de l'échantillon à analyser, divisé par le volume total de l'espace interne.

18. Procédé de contrôle microbiologique selon l'une quelconque des revendications 15 à 17, **caractérisé en ce que** l'étape de dépressurisation abaisse la pression gazeuse absolue à l'intérieur de l'espace interne fermé (12), ramenée à une température de 25°C, à une valeur strictement inférieure à 60 kPa absolus, de préférence strictement inférieure à 30 kPa absolus, plus préférentiellement strictement inférieure à 20 kPa absolus.

19. Utilisation d'un dispositif de contrôle microbiologique tel que revendiqué par l'une des revendications 1 à 14 dans un procédé de contrôle d'un liquide à analyser susceptible de contenir au moins un micro-organisme.

20. Utilisation selon la revendication 19, **caractérisée en ce qu'**elle comporte les étapes consistant à :

- raccorder un contenant de liquide à analyser au port d'entrée (40) ;
- ouvrir l'obturateur (46) du port d'entrée (40) pour permettre le passage du liquide à analyser du récipient vers l'espace interne fermé (12).

21. Utilisation selon la revendication 20, **caractérisée en ce qu'**elle comporte les étapes ultérieures consistant à :

- refermer l'obturateur (46) du port d'entrée (40) ;
- déconnecter le contenant de liquide à analyser ;
- faire incuber, dans le dispositif de contrôle microbiologique (10), un éventuel micro-organisme contenu initialement dans le liquide à analyser.

22. Utilisation selon la revendication 21, **caractérisée en ce qu'**elle comporte une étape ultérieure consistant à détecter, dénombrer, identifier et/ou caractériser visuellement un éventuel micro-organisme contenu initialement dans le liquide à analyser par vision au travers d'une portion transparente de l'enceinte du dispositif de contrôle microbiologique (10).

**Patentansprüche**

1. Mikrobiologische Testvorrichtung (10) zum Testen einer zu analysierenden Flüssigkeit, die mindestens einen Mikroorganismus enthalten kann, in einer Konfiguration zur Bereitstellung der mikrobiologischen Testvorrichtung (10) vor der Verwendung und die Folgendes umfasst:

- einen geschlossenen Innenraum (12), der von einer Kammer begrenzt wird und zur Aufnahme der zu analysierenden Flüssigkeit vorgesehen ist;
- ein mikrobiologisches Filtrationsmittel (32), das im geschlossenen Innenraum (12) angeordnet ist und im Innenraum ein erstes Kompartiment (12a) von einem zweiten Kompartiment (12b) des geschlossenen Innenraums trennt;
- eine Einlassöffnung (40) für die zu analysierende Flüssigkeit, wobei die Einlassöffnung in das erste Kompartiment (12a) des geschlossenen Innenraums einmündet,

**dadurch gekennzeichnet, dass** die mikrobiologische Testvorrichtung (10) im Inneren des geschlossenen Innenraums eine Nährstoffschicht (36) umfasst, die eine Zusammensetzung eines mikrobiologischen Kulturmediums umfasst, wobei die Nährstoffschicht (32) sich in Kontakt mit dem Filtrationsmittel (32) befindet, und dadurch, dass die Einlassöffnung (40) der mikrobiologischen Testvorrichtung ein Verschlussorgan (46) umfasst,

und dadurch, dass:

- sich das Verschlussorgan (46) der Einlassöffnung (40) in einem geschlossenen Zustand befindet, um die Einlassöffnung (40) und den geschlossenen Innenraum (12) luftdicht zu verschließen;
- der absolute Gasdruck im Inneren des geschlossenen Innenraums (12), der auf eine Temperatur von 25 °C bezogen ist, streng kleiner als der Standardatmosphärendruck von 100 kPa bei 25 °C ist, sodass die Vorrichtung dazu fähig ist, bei einem ersten Öffnen des Verschlussorgans (46) eine Ansaugung durch die Einlassöffnung zu erzeugen,
- der geschlossene Innenraum (12) gegen jegliche externe Saugquelle isoliert ist.

2. Mikrobiologische Testvorrichtung nach dem vorhergehenden Anspruch, wobei das mikrobiologische Kulturmedium der Nährstoffschicht (36) dehydratisiert ist.

3. Mikrobiologische Testvorrichtung nach einem der vorhergehenden Ansprüche, **dadurch gekennzeichnet, dass** die mikrobiologische Testvorrichtung (10) einen Träger (48) für das Filtrationsmittel (32) und die Nährstoffschicht (36) umfasst.

4. Mikrobiologische Testvorrichtung nach Anspruch 3, **dadurch gekennzeichnet, dass** die Nährstoffschicht (36) zwischen dem Filtrationsmittel (32) und dem Träger (48) für das Filtrationsmittel (32) örtlich fixiert ist.

5. Mikrobiologische Testvorrichtung nach einem der Ansprüche 3 oder 4, **dadurch gekennzeichnet, dass** der Träger (48) für das Filtrationsmittel (32) tragende Trennwände (50) umfasst, die im zweiten Kompartiment (12b) angeordnet sind.

6. Mikrobiologische Testvorrichtung nach einem der Ansprüche 3 oder 4, **dadurch gekennzeichnet, dass** der Träger (48) für das Filtrationsmittel (32) eine Lochscheibe (49) umfasst, die sich quer durch den geschlossenen Innenraum zwischen dem ersten Kompartiment (12a) und dem zweiten Kompartiment (12b) erstreckt.

7. Mikrobiologische Testvorrichtung nach einem der vorhergehenden Ansprüche, **dadurch gekennzeichnet, dass** ein wasserabsorbierendes Material im zweiten Kompartiment (12b) angeordnet ist.

8. Mikrobiologische Testvorrichtung nach einem der vorhergehenden Ansprüche, **dadurch gekennzeichnet, dass** das Verschlussorgan (46) der Einlassöffnung (40) ein Ventil umfasst.

9. Mikrobiologische Testvorrichtung nach einem der vorhergehenden Ansprüche, **dadurch gekennzeichnet, dass** die Kammer der mikrobiologischen Testvorrichtung mindestens einen Hauptkörper (14) umfasst, der zumindest einen Teil des zweiten Kompartiments (12b) begrenzt, und eine Abdeckung (16) umfasst, die zumindest einen Teil des ersten Kompartiments (12a) begrenzt, wobei der Hauptkörper und die Abdeckung als getrennte Teile gebildet sind, die miteinander verbunden sind, wodurch die mikrobiologische Testvorrichtung gebildet wird.

10. Mikrobiologische Testvorrichtung nach einem der vorhergehenden Ansprüche, **dadurch gekennzeichnet, dass** die Kammer der mikrobiologischen Testvorrichtung mindestens einen transparenten Teil umfasst.

11. Mikrobiologische Testvorrichtung nach einem der vorhergehenden Ansprüche, **dadurch gekennzeichnet, dass** die Einlassöffnung (40) einen Verteiler (46) umfasst, der mehrere getrennte Durchlässe für die zu analysierende Flüssigkeit umfasst.

12. Mikrobiologische Testvorrichtung nach einem der vorhergehenden Ansprüche, **dadurch gekennzeichnet, dass** in der Konfiguration zur Bereitstellung der mikrobiologischen Testvorrichtung (10) vor der Verwendung der absolute Gasdruck im Inneren des geschlossenen Innenraums (12) derart ist, dass er beim Zulauf eines vorbestimmten Volumens der zu analysierenden Probe den Zulauf eines vorbestimmten Volumens der zu analysierenden Probe ohne eine Abführung von Fluid aus dem Innenraum ermöglicht.

13. Mikrobiologische Testvorrichtung nach einem der vorhergehenden Ansprüche, **dadurch gekennzeichnet, dass** in der Konfiguration zur Bereitstellung der mikrobiologischen Testvorrichtung (10) vor der Verwendung der absolute Gasdruck im Inneren des geschlossenen Innenraums (12) streng kleiner als der Standardatmosphärendruck multipliziert mit dem Verhältnis des endgültigen freien Volumens im Innenraum nach dem Zulauf eines vorbestimmten Volumens der zu analysierenden Probe dividiert durch das Gesamtvolumen des Innenraums ist.

14. Mikrobiologische Testvorrichtung nach einem der vorhergehenden Ansprüche, **dadurch gekennzeichnet, dass** in der Konfiguration zur Bereitstellung der mikrobiologischen Testvorrichtung (10) vor der Verwendung der absolute Gasdruck im Inneren des geschlossenen Innenraums (12), der auf eine Temperatur von 25 °C bezogen ist, streng kleiner als 60 kPa absolut, vorzugsweise streng kleiner als 30 kPa absolut, noch mehr bevorzugt streng kleiner als 20 kPa absolut ist.

15. Verfahren zur Bereitstellung einer mikrobiologischen Testvorrichtung nach einem der vorhergehenden Ansprüche und die so ausgebildet ist, dass sie eine zu analysierende Flüssigkeit testet, die mindestens einen Mikroorganismus enthalten kann, das die Bereitstellung einer mikrobiologischen Testvorrichtung (10) umfasst, **dadurch gekennzeichnet, dass** das Verfahren die Bereitstellung einer Nährstoffschicht (32) umfasst, die im Inneren des geschlossenen Innenraums (12) aufgenommen wird und eine Zusammensetzung eines mikrobiologischen Kulturmediums umfasst, wobei sich die Nährstoffschicht (36) in Kontakt mit dem Filtrationsmittel (32) befindet, und dadurch, dass das Verfahren vor jeglicher Verbindung der mikrobiologischen Testvorrichtung (10) mit einem Behälter der zu analysierenden Flüssigkeit nacheinander und in dieser Reihenfolge Folgendes umfasst:

    - einen Druckentlastungsschritt zum Vermindern des absoluten Gasdrucks im Inneren des geschlossenen Innenraums (12), bezogen auf eine Temperatur von 25 °C, auf streng weniger auf 100 kPa;
    - einen Verschlussschritt zum luftdichten Verschließen des geschlossenen Innenraums (12).

16. Verfahren nach Anspruch 15, **dadurch gekennzeichnet, dass** beim Druckentlastungsschritt der absolute Gasdruck im Inneren des geschlossenen Innenraums (12) auf einen solchen Wert gesenkt wird, dass er beim Zulauf eines vorbestimmten Volumens der zu analysierenden Probe den Zulauf eines vorbestimmten Volumens der zu analysierenden Probe ohne eine Abführung von Fluid aus dem Innenraum ermöglicht.

17. Mikrobiologisches Testverfahren nach einem der Ansprüche 15 oder 16, **dadurch gekennzeichnet, dass** beim Druckentlastungsschritt der absolute Gasdruck im Inneren des geschlossenen Innenraums (12) auf einen Wert vermindert wird, der streng kleiner als der Standardatmosphärendruck multipliziert mit dem Verhältnis des endgültigen freien Volumens im Innenraum nach dem Zulauf eines vorbestimmten Volumens der zu analysierenden Probe dividiert durch das Gesamtvolumen des Innenraums ist.

18. Mikrobiologisches Testverfahren nach einem der Ansprüche 15 bis 17, **dadurch gekennzeichnet, dass** beim Druckentlastungsschritt der absolute Gasdruck im Inneren des geschlossenen Innenraums (12), der auf eine Temperatur von 25 °C bezogen ist, auf einen Wert vermindert wird, der streng kleiner als 60 kPa absolut, vorzugsweise streng kleiner als 30 kPa absolut, noch mehr bevorzugt streng kleiner als 20 kPa absolut ist.

19. Verwendung einer mikrobiologischen Testvorrichtung wie in einem der Ansprüche 1 bis 14 beansprucht in einem Verfahren zum Testen einer zu analysierenden Flüssigkeit, die mindestens einen Mikroorganismus enthalten kann.

20. Verwendung nach Anspruch 19, **dadurch gekennzeichnet, dass** sie die Schritte umfasst, die aus Folgendem bestehen:

    - dem Verbinden eines Behälters der zu analysierenden Flüssigkeit mit der Einlassöffnung (40);
    - dem Öffnen des Verschlussorgans (46) der Einlassöffnung (40), um den Durchlass der zu analysierenden Flüssigkeit vom Behälter zum geschlossenen Innenraum (12) zu ermöglichen.

21. Verwendung nach Anspruch 20, **dadurch gekennzeichnet, dass** sie die weiteren Schritte umfasst, die aus Folgendem bestehen:

    - dem Wiederverschließen des Verschlussorgans (46) der Einlassöffnung (40);
    - dem Trennen des Behälters der zu analysierenden Flüssigkeit;
    - dem Inkubieren in der mikrobiologischen Testvorrichtung (10) eines etwaigen ursprünglich in der zu analysierenden Flüssigkeit enthaltenen Mikroorganismus.

22. Verwendung nach Anspruch 21, **dadurch gekennzeichnet, dass** sie einen weiteren Schritt umfasst, der aus einem visuellen Detektieren, Zählen, Identifizieren und/oder Charakterisieren eines etwaigen ursprünglich in der zu analysierenden Flüssigkeit enthaltenen Mikroorganismus durch ein Betrachten quer durch einen transparenten Abschnitt der Kammer der mikrobiologischen Testvorrichtung (10) besteht.

# Claims

1. Microbiological testing device (10) for testing a liquid to be analysed that is liable to contain at least one microorganism, in a configuration for provision of the microbiological testing device (10) before use, and comprising:

   - a closed inner space (12) delimited by a chamber and intended to receive the liquid to be analysed;
   - a microbiological filtration means (32) arranged in the closed inner space (12) and separating, in the closed inner space, a first compartment (12a) from a second compartment (12b) of the closed inner space;
   - an inlet port (40) for the liquid to be analysed, the inlet port opening into the first compartment (12a) of the closed inner space,

   **characterized in that** the microbiological testing device (10) comprises, inside the closed inner space, a nutrient layer (36) comprising a composition of a microbiological culture medium, the nutrient layer (32) being in contact with the filtration means (32), **in that** the inlet port (40) of the microbiological testing device comprises an open/close member (46), and **in that**:

   - the open/close member (46) of the inlet port (40) is in a closed state for closing the inlet port (40) and the closed inner space (12) in an airtight manner;
   - the absolute gas pressure inside the closed inner space (12), relative to a temperature of 25°C, is strictly less than the standard atmospheric pressure of 100 kPa at 25°C, such that the device is able to create suction through the inlet port during a first opening of the open/close member (46),
   - the closed inner space (12) being isolated from any external source of suction.

2. Microbiological testing device according to the preceding claim, in which the microbiological culture medium of the nutrient layer (36) is dehydrated.

3. Microbiological testing device according to either of the preceding claims, **characterized in that** the microbiological testing device (10) comprises a support (48) for the filtration means (32) and the nutrient layer (36).

4. Microbiological testing device according to Claim 3, **characterized in that** the nutrient layer (36) is locally clamped between the filtration means (32) and the support (48) for the filtration means (32).

5. Microbiological testing device according to either of Claims 3 and 4, **characterized in that** the support (48) for the filtration means (32) comprises support partitions (50) arranged in the second compartment (12b).

6. Microbiological testing device according to either of Claims 3 and 4, **characterized in that** the support (48) for the filtration means (32) comprises a screen (49) which extends across the closed inner space between the first compartment (12a) and the second compartment (12b).

7. Microbiological testing device according to any one of the preceding claims, **characterized in that** a water-absorbing material is arranged in the second compartment (12b).

8. Microbiological testing device according to any one of the preceding claims, **characterized in that** the open/close member (46) of the inlet port (40) comprises a valve.

9. Microbiological testing device according to any one of the preceding claims, **characterized in that** the chamber of the microbiological testing device comprises at least one main body (14) which at least partially delimits the second compartment (12b), and comprises a cover (16) which at least partially delimits the first compartment (12a), the main body and the cover being formed of separate parts assembled together to form the microbiological testing device.

10. Microbiological testing device according to any one of the preceding claims, **characterized in that** the chamber of the microbiological testing device comprises at least one transparent portion.

11. Microbiological testing device according to any one of the preceding claims, **characterized in that** the inlet port (40) comprises a distributor (46) comprising several separate passages for the liquid to be analysed.

12. Microbiological testing device according to any one of the preceding claims, **characterized in that**, in the configuration for provision of the microbiological testing device (10) before use, the absolute gas pressure inside the closed inner space (12) is such that it enables the ingress of a predetermined volume of the sample to be analysed without discharging fluid from the inner space during the ingress of a predetermined volume of the sample to be analysed.

13. Microbiological testing device according to any one of the preceding claims, **characterized in that**, in the configuration for provision of the microbiological testing device (10) before use, the absolute gas pressure inside the closed inner space (12) is strictly less than the standard atmospheric pressure multiplied by the ratio of the final free volume in the inner space, after the ingress of a predetermined volume of the sample to be analysed, divided by the total volume of the inner space.

14. Microbiological testing device according to any one of the preceding claims, **characterized in that**, in the configuration for provision of the microbiological testing device (10) before use, the absolute gas pressure inside the closed inner space (12), relative to a temperature of 25°C, is strictly less than 60 kPa absolute, preferably strictly less than 30 kPa absolute, more preferentially strictly less than 20 kPa absolute.

15. Process for providing a microbiological testing device according to one of the preceding claims and configured to test a liquid to be analysed that is liable to contain at least one microorganism, involving providing a microbiological testing device (10), **characterized in that** the process comprises the provision of a nutrient layer (32) received inside the closed inner space (12) and comprising a composition of a microbiological culture medium, the nutrient layer (36) being in contact with the filtration means (32), and **in that** the process comprises, before any connection of the microbiological testing device (10) to a container of liquid to be analysed, successively and in this order:

- a step of depressurization to lower the absolute gas pressure inside the closed inner space (12), relative to a temperature of 25°C, to strictly less than 100 kPa;
- a step of closure to close the closed inner space (12) in an airtight manner.

16. Process according to Claim 15, **characterized in that** the depressurization step lowers the absolute gas pressure inside the closed inner space (12) to a value such that it enables the ingress of a predetermined volume of the sample to be analysed without discharging fluid from the inner space during the ingress of a predetermined volume of the sample to be analysed.

17. Microbiological testing process according to either of Claims 15 and 16, **characterized in that** the depressurization step lowers the absolute gas pressure inside the closed inner space (12) to a value strictly less than the standard atmospheric pressure multiplied by the ratio of the final free volume in the inner space, after the ingress of a predetermined volume of the sample to be analysed, divided by the total volume of the inner space.

18. Microbiological testing process according to any one of Claims 15 to 17, **characterized in that** the depressurization step lowers the absolute gas pressure inside the closed inner space (12), relative to a temperature of 25°C, to a value strictly less than 60 kPa absolute, preferably strictly less than 30 kPa absolute, more preferentially strictly less than 20 kPa absolute.

19. Use of a microbiological testing device according to one of Claims 1 to 14 in a process for testing a liquid to be analysed that is liable to contain at least one microorganism.

20. Use according to Claim 19, **characterized in that** it comprises the steps consisting in:

- connecting a container of liquid to be analysed to the inlet port (40);
- opening the open/close member (46) of the inlet port (40) to enable the passage of the liquid to be analysed from the receptacle towards the closed inner space (12).

21. Use according to Claim 20, **characterized in that** it comprises the subsequent steps consisting in:

- closing the open/close member (46) of the inlet port (40);
- disconnecting the container of liquid to be analysed;
- incubating, in the microbiological testing device (10), a microorganism potentially initially contained in the liquid to be analysed.

22. Use according to Claim 21, **characterized in that** it comprises a subsequent step consisting in visually detecting, counting, identifying and/or characterizing a microorganism potentially initially contained in the liquid to be analysed by viewing through a transparent portion of the chamber of the microbiological testing device (10).

FIG.1

FIG.2

FIG.3

FIG.4

FIG.5

FIG.6

FIG.7

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2014342447 A1

- EP 1783494 A